# EUROPEAN PATENT SPECIFICATION

(11) **EP 1 622 589 B1**
(45) Date of publication and mention of the grant of the patent: **15.03.2017**
(21) Application number: 03816832.4
(22) Date of filing: 08.12.2003
(51) Int. Cl.: A61K 31/167, A61K 31/195, A61K 38/48, A61P 27/06

(54) **COMPOSITIONS, TARGETS, METHODS AND DEVICES FOR THE THERAPY OF OCULAR AND PERIOCULAR DISORDERS**
ZUSAMMENSETZUNGEN, TARGETS, VERFAHREN UND GERÄTE ZUR BEHANDLUNG VON OKULAREN UND PERIOKULAREN ERKRANKUNGEN
COMPOSITIONS, CIBLES, METHODES ET DISPOSITIFS POUR LE TRAITEMENT DE TROUBLES OCULAIRES ET PERI-OCULAIRES

(30) Priority: 24.04.2003 US 421956
(43) Date of publication of application: 08.02.2006
(73) Proprietor: Abreu, Marcio Marc, North Haven, CT 06473 (US)
(72) Inventor: Abreu, Marcio Marc, North Haven, CT 06473 (US)
(74) Representative: Pfenning, Meinig & Partner mbB
(86) International application number: PCT/US2003/038740
(87) International publication number: WO 2004/096183

(56) References cited:
- WO-A-00/28999
- WO-A-01/42191
- WO-A-98/43612
- US-A- 6 090 847
- US-A- 6 117 912
- US-B1- 6 306 403
- US-B1- 6 350 781
- US-B1- 6 403 649
- US-B1- 6 545 045
- KIKKAWA, DON O ET AL: "Botulinum A Toxin Injection for Restrictive Myopathy of Thyroid-related Orbitopathy: Effects on Intraocular Pressure" AMERICAN JOURNAL OF OPHTHALMOLOGY, vol. 135, no. 4, April 2003 (2003-04), pages 427-431, XP002529635 ISSN: 0002-9394
- LEV R ET AL: "Prophylactic lidocaine use preintubation: A review" JOURNAL OF EMERGENCY MEDICINE, PERGAMON PRESS, NEW YORK, NY, GB, vol. 12, no. 4, 1 July 1994 (1994-07-01), pages 499-506, XP023254426 ISSN: 0736-4679 [retrieved on 1994-07-01]
- WEBER ET AL: "Neovascular glaucoma. Current management" SURVEY OF OPHTHALMOLOGY, SURVEY OF OPHTHALMOLOGY INC, XX, vol. 26, no. 3, 1 November 1981 (1981-11-01), pages 149-153, XP023265241 ISSN: 0039-6257 [retrieved on 1981-11-01]

## Description

### FIELD OF THE INVENTION

The present invention includes compositions for treating ocular, periocular and facial abnormalities by acting on muscle activity

### BACKGROUND OF THE INVENTION

Glaucoma is a leading cause of blindness worldwide characterized by decreased filtration of eye fluid and increase of intraocular pressure to values which the eye cannot withstand. Treatment of glaucoma and other eye disorders such as ocular hypertension, retinal vein occlusion, ischemic optic neuropathy and the like includes the management of intraocular pressure. Currently, the main approach for controlling intraocular pressure is by using eye drops and some times pills. All of the current medications act by reducing internal pressure effects pressure effects inside the eye).

The eye can be considered as a balloon filled with water. Internal pressure effects consist of factors inside the eye that cause change in eye pressure. The two internal factors are eye fluid production and eye fluid drainage, with said factors being present inside the eye. External pressure effects consists of factors outside the eye, i.e., outside the balloon (eye), but that causes change in pressure inside the eye.

Unfortunately today doctors can only choose drugs to reduce internal pressure' effects, since current treatment methods comprise of reducing production of eye fluid (e.g.: beta blockers) and/or increasing the drainage of eye fluid (e.g.: prostaglandin analogs). However, glaucomatous damage is not only due to internal pressure effects, but also due to external pressure effects (pressure effects outside the eye). Increased eye pressure, eye pressure spikes and fluctuation of pressure due to external pressure effects such as blinking, forceful closure of the eyelid, and eyelid muscle tension can cause progressive damage to the optic nerve. Because of the lack of symptoms caused by those external pressure effects there is gradual and painless loss of vision. As a result, millions of glaucoma victims are unaware that they have external pressure effects causing increase in eye pressure and face eventual blindness if those external pressure effects remain undetected and untreated. Although the eyelid muscles are not hyperactive in glaucoma, because the drainage system does not function properly, external pressure by muscles acting on the eye cause increased and sustained pressure, which can lead to damage and blindness.

It is known in the prior art that certain drugs such as botulinum toxin can be used to treat a variety of neuromuscular disorders related to hypertonicity and spasticity. Those drugs have been used to treat muscle hyperactivity and muscle overcontraction. The prior art has used those agents to treat muscles which had abnormal function, characterized by hyperactive neuromuscular activity in striated or smooth muscle, involuntary muscle contractions and muscle spasm. Botulinum toxin was injected directly into the hyperactive or hypertonic muscle or in the surrounding area of said hyperactive or hypertonic muscle. Alternatively, some agents such as clonazepan were taken by mouth to reduce the hypertonicity. Those agents have been used in a variety of muscle disorders in order to reduce the excess muscle contraction and sphincter contraction. Botulinum toxin in particular have been used for the treatment of many hyperactive muscle disorders. The intramuscular injection of botulinum toxin has been used to treat, blepharospasm, strabismus, hemifacial spasm, oral mandibular dystonia, limb dystonias, cervical dystonia, myofacial pain, achalasia, spastic disorders, juvenile cerebral palsy, focal dystonias, tension headache and spasticity.

For the treatment of blepharospasm and hemifacial spasm injections of botulinum toxin are done at multiple sites in the eyelid and facial musculature to decrease the spastic state or hyperactive state of said muscles. Botulinum toxin has been used to treat many disorders in which a muscle is hyperactive, but botulinum toxin has not been used to lower eye pressure, to reduce external pressure effects, to increase blood flow to the eye and to treat glaucoma.

Botulinum toxin has also been used to treat muscle overaction (e.g.: muscle contraction causing wrinkles). The cosmetic treatment to reduce wrinkles using botulinum toxin is very expensive, in addition to being painful. There is a need to identify a method and compound that can reduce or eliminate facial wrinkles that is painless and low cost.

There is further a need to identify a method, biological targets and compounds that can effectively treat the external pressure effects acting on the eye, thereby alleviating said external pressure effects on said eye. There is also a need to treat completely normal eye muscles that despite being non-hyperactive are causing damage to the eye and blindness.

There is yet a need to bring said completely normal eye muscles and facial muscles to a lower reactive state, in a manner so as to eliminate the damaging external pressure effects and glaucomatous changes caused by said eye muscles and facial muscles. In addition, there is a need to accomplish all of the above tasks while preserving normal eyelid muscle function. There is yet a need to achieve all therapeutic benefits while enhancing efficacy of glaucoma eye drops such as prostaglandin analogues.
Kikkawa, D.O. et al. (American Journal of Ophthalmology, April 2003, vol. 135, no. 4, p. 427-431) teach that botulinium A toxin injections cause a secondary effect to lower intraocular pressure (IOP) in patients with restrictive strabismus associated with thyroid-related orbitopathy. Weber, P.A. (Survey of Ophthalmology, November 1981, vol. 26, no. 3, p. 149-153) teaches a retrobulbar injection of alcohol and lidocaine for reducing IOP in patients with glaucoma.

### SUMMARY OF THE INVENTION

The present invention meets the needs of the prior art and provides compositions for effectively treating the external pressure effects acting on the eye or around the eye by the administration of compounds that reduce and/or modulate said external pressure effects and achieve long lasting reduction of: pressure fluctuation, pressure spikes and baseline eye pressure while preserving normal eye muscle function.

The invention relates to chemical compounds and compositions acting on external pressure effects for the therapy of ocular and periocular disorders including pressure effects by the eyelids, eye muscles, and facial musculature. More particularly, the invention is concerned with the use of compositions such as botulinum toxin and lidocaine and alcohol for modulating
blinking and facial musculature and eye muscles for the treatment of glaucoma. Specifically, the invention proposes an effective amount of a compound that reduces external pressure effects for use in treating glaucoma in a mammal, wherein external pressure effects experienced by the mammal are alleviated, wherein said compound is botulinum toxin or a combination of lidocaine and alcohol and wherein the compound is administered by percutaneous injection into one or more points in the musculature at the eyelid margin adjacent to the muscle of Riolan.

The description also includes methods for treating patients with eye disorders including the step of measuring the force of the eyelid or the pressure caused by the eyelid, and then the step of delivering medication based upon the measurements of the first step.

The description also includes drug delivery systems and routes of administration used to deliver the compound to treat said eye disorders. The chemical compounds are used to modulate or reduce the force of muscles for the therapy of glaucoma, diabetic retinopathy, thyroid eye disorder, ocular vascular abnormalities, macular edema, macular degeneration, optic neuritis and ischemic optic neuropathy.

The description further discloses means to reduce muscle overcontraction and reduce facial wrinkles.

The description yet discloses means to reduce muscle contraction during and after surgical procedures such as refractive surgery and cataract surgery including a method comprising the step of measuring eyelid muscle function and then applying medication to control said eyelid muscle function in order to enhance the outcome of the surgery.

All of the disorders that can benefit from reduction of eye pressure can be treated by administering a compound in a concentration effective to reduce external pressure effects including eyelid muscle activity and blinking, thereby reducing eye pressure spikes and eye pressure fluctuation with consequent increase in perfusion pressure and blood flow to the eye. All of the disorders that can benefit from reduction of eye pressure and/or enhancement of blood flow can be treated by using the compounds and methods disclosed herein.

When glaucoma was first understood as a pathological entity, as in the earlier writings of Mackenzie in 1830, glaucoma was primarily seen as a disease of excess fluid production. It was not until about 30 years later that the work of Weber and Knies indicated that the increase in eye pressure was related to decreased drainage. The two physical variables, aqueous inflow and outflow, causes an internal pressure effect. Those two factors, aqueous inflow and outflow, are the only current factors used to design therapeutic approaches for eye pressure lowering to prevent glaucomatous damage.

Despite treating internal pressure effects with the use of eye drops which decrease aqueous inflow and increase aqueous outflow, patients are still going blind. There are unknown factors in glaucoma that are causing blindness:
a. eye pressure measured in the office has been acceptable but despite that visual field is progressing, meaning it is worse than before;
b. eye pressure measured at the office visit has been acceptable but despite that there is damage to the optic nerve;
c. normal and even low eye pressure measured in the office have been associated with damage to the optic nerve and lost vision; and
d. patients went blind due to glaucoma despite normal and even low eye pressure measured in the office.

Careful studies led to the discovery that glaucomatous damage and blindness is also due to external pressure effects. Meticulous studies led to the therapeutic approaches of the present invention and to the identification and measurement of external pressure effects.

External pressure effects (EPE) are physical factors that are external to the eye but that affects the pressure inside the eye. EPE can cause an increase in intraocular (IOP) and contribute to blindness despite normal IOP and despite the use of drugs to treat internal pressure effects. The external physical events in the eye that can increase IOP, create substantial pressure spikes, augment eye pressure fluctuation and lead to visual loss are:
1) Blinking: the eye pressure can increase by 15 mm Hg or more with every blink. Studies identified that ordinary blink can increase eye pressure over 200% compared to the baseline.
2) Squeezing: the eye pressure can increase by 90 mm Hg or more with every squeezing of the eyelids. Studies identified that ordinary squeezing the eye can increase eye pressure over 700% compared to the baseline.
3) Eyelid muscle tension can increase baseline eye pressure by 5 to 10 mm Hg or more. Studies identified that the eyelid at rest can increase eye pressure over 50 to 100% compared to the baseline.

In glaucoma patients the drainage system is impaired and there is a higher increase and sustained increase in eye pressure during blinking or with squeezing the eyelids or due to muscle tension. Normal individuals can recover from this eye pressure increase in a more rapid fashion by increasing drainage of eye fluid. However, in glaucoma patients the drainage system is impaired and such increase in eye pressure during blinking or with squeezing the eyelids or due to muscle tension remains high, which can lead to damage to the optic nerve and eventually damage to the optic nerve. This is expected since the drainage system in glaucoma does not function adequately. The present invention discovered that administration of a neurotoxin or other compounds according to the principles of the present invention provides significant alleviation of the external pressure effects and that those compounds can be used for treating glaucoma and other conditions that can benefit from eye pressure modulation or eye pressure reduction and enhancement of blood flow.

EPE is any physical factor that is not inside the eye but that affects the pressure insides the eye and include muscle action, muscle tension, muscle size, ligaments around the eye, cartilage around the eye, fibrous tissue around the eye, skin around the eye, tarsal plates in the eyelids, sclera, conjunctiva and the like as well as external pressure effects caused by compression by objects outside the eye including pressing the eye against a physical object. Muscle action includes any motion by the muscle that affects eye pressure such as blinking or squeezing the eyelids. Muscle tension includes any effect by the muscle at rest that affects eye pressure. Eye pressure is herein used interchangeably with IOP and denotes pressure inside the eye.

The increase in eye pressure due to Blinking, Squeezing and Muscle Tension causes significant and frequent increase in eye pressure, is an important contributory factor for damaging the eye, and can lead to visual loss in many disorders.

Blinking is one of the most frequently performed muscle actions and the force of blinking cannot be controlled voluntarily. Involuntary blinking occurs usually every 2 to 10 seconds. If a patient has glaucoma it could mean that the eye could have been hammered an average of 16,000 times on a daily basis with pressures 15 mm Hg above the baseline which would lead to great damage to the eye over time. Ordinary blink increases eye pressure by 50% to over 200% compared to baseline. The compositions of the present invention modulate and reduce muscle action during blinking to prevent excessive increase of eye pressure while maintaining adequate blinking function.

Squeezing the eyes: Over 14 daily conditions lead to squeezing the eyelids including any strong emotion, practice of sports, showering, concentrating, lifting weights, intense sun light, smelling something odious, waking up, tiredness, sleeping and dreaming. Force of squeezing cannot be totally controlled voluntarily and cannot be controlled during sleeping. If a patient has glaucoma it could mean that the eye could have been hammered an average of 14 times on a daily basis with pressures over 100 mm Hg which would lead to great damage to the eye in glaucoma patients. Even normal individuals can sustain damage at this high level of pressure. The compositions of the present invention modulate muscle action to prevent excessive increase of eye pressure during squeezing the eyes while maintaining adequate eyelid squeezing function.

Eyelid muscle tension: external pressure by the eyelid at rest sometimes can be so intense to the point of creating optical changes and changing the configuration of the cornea including change in the axis of the astigmatism and the Venetian Blind Phenomenon. This pressure effect can lead to a 50% increased eye pressure in glaucoma patients and damage to the eye since the drainage system is not working properly. The compositions of the present invention modulate lid muscle tension to prevent increase of eye pressure while maintaining adequate eyelid function.

Even normal people without glaucoma, but who have excessive and forceful blinking, such as in hemifacial spasm, became glaucomatous and went blind. Excess of muscle contraction in one eye causing substantial increase of EPE such as in blepharospasm leads to loss of vision in the eye affected by EPE only. If people without glaucoma can become glaucomatous and can go blind, glaucoma patients are at a much higher risk of blindness because glaucomatous eyes cannot tolerate pressure effects like normal eyes can, and glaucoma patients can sustain damage at much lower levels of eye pressure.

Glaucomatous eyes cannot tolerate EPE like normal eyes and glaucoma patients can sustain irreparable damage at much lower pressure levels. Small changes of pressure above Target Pressure can lead to visual lass in glaucoma. Target Pressure is referred to herein as the target IOP for therapy, thus any IOP value above the Target Pressure can lead to lass of visual function.

Blinking and squeezing the eyes is natural and universal activity and glaucoma patients, diabetics, patients with retinal vascular occlusions, macular disease, ischemic optic neuropathy, and the like are at risk of visual loss due to EPE when said patients blink or squeeze their eyes or due to muscle tension at rest. EPE can lead to IOP well above the Target Pressure and glaucoma patients can sustain irreparable damage to their eyes if the EPE remain untreated.

Several aspects are provided by the present invention including treating glaucoma with an agent that decreases or modulates an external pressure effect. Another aspect is to physically achieve a therapeutic effect of eye pressure lowering by acting on external pressure effects. A further aspect is to achieve a therapeutic effect while preserving normal eye muscle function.

Another aspect of the present invention features modulating muscle tension and modulating muscle action including modulating blinking or decreasing the ability to squeeze the eyelids which promote a reduction in EPE and decrease in baseline eye pressure, a decrease in pressure spikes and a reduction in the fluctuation of eye pressure. The therapeutic effect is achieved by using a chemomodulating agent such as botulinum toxin or a composition of alcohol and lidocaine. The modulation of activity of the neuromuscular junction or decrease in muscular activity of eye muscles, eyelid muscles and facial muscles can be used to control and
reduce spikes of eye pressure, baseline eye pressure and eye pressure fluctuation.

Experiments have demonstrated that reduction of eye pressure can be achieved by decreasing muscular activity of certain eyelid muscles.

The discovery by the present invention of the increase of eye pressure by eyelid muscle tension, blinking and squeezing of the eyelids associated with the discovery that some compounds can modulate and/or decrease external pressure effects such as blinking and the spike of eye pressure with subsequent preservation of sight have lead to the development of various novel methods and drugs for the treatment of various eye disorders including glaucoma, ocular hypertension, diabetic retinopathy, retinal vascular occlusions, ischemic optic neuropathy, macular degeneration and any condition that can benefit from decreased eye pressure or increased blood flow. The description provides methods for reducing eye pressure by selectively and temporarily inactivating muscles or muscle groups responsible for blinking and closure of the eye including involuntary and forceful blinking and squeezing the eyelids. The present invention includes percutaneous injection of a compound into one or more points in the musculature at the eyelid margin adjacent to the muscle of Riolan, with said agent capable of temporarily blocking muscle activity. The effect of the treatment is to decrease the force of the lid muscle and/or blinking and/or to reduce forceful closure of the eye, which causes pressure spikes. Using the procedure disclosed herein, damage by eye pressure spikes and eye pressure fluctuation can be eliminated by decreasing such enormous range of pressure fluctuation and spikes throughout the day and night caused by the external pressure effects.

The compositions of the present invention cannot be substituted by any existing eye drops. Whatever sight-preserving eye drops a patient uses, he/she would benefit from an additional decrease in eye pressure from the compositions of the present invention Furthermore, the targets hit by the compositions of the present invention prevent eye pressure spikes and eye pressure fluctuation. No eye drops hit those targets and no current eye drop can reduce the blinding effects due to external pressure. A n improved eye pressure lowering effect of prostaglandin analogs was observed when used in conjunction with the compositions of the present invention.

The compositions of the present invention give more physical stability to the eye, and combining conventional eye drops to treat glaucoma with said compositions enhance the therapeutic effect of said eye drops. The present description also provides methods to enhance the effect of prostaglandin analogues including Lumigan (available from Allergan, Irvine; CA), Rescula (available from Novartis Ophthalmics, Duluth, GA), Travatan (available from Alcon, Fort Worth, TX) and ( Xalatan (available from Pharmacia, Peacock, NJ).

A variety of devices by Abreu (U.S. Pat. No. 5,830,139, U.S. Pat. No. 6,120,460, U.S. Pat. No. 6,123,668, U.S. Pat. No. 6,213,943, U.S. Pat. No. 6,312,393) to monitor intraocular pressure can monitor and identify the increase in eye pressure caused by external pressure effects. Besides the devices by Abreu, any device capable of measuring the aforementioned EPE including at least one of devices for measuring IOP increase by muscle activity, force of the muscle, force of the eyelid, speed of contraction, elasticity of the muscles, eyelid distractibility and eyelid laxity, can be used to measure EPE. Collectively all the devices measuring EPE are referred to herein as Lid Measuring Devices (LMD).

The methods of the present description includes measurement of pressure, force and the like by the eye muscles using LMD including any device to measure the aforementioned external pressure effects or the pressure increase caused by muscles or the force of the muscles or the force of the eyelid. The method more specifically includes the step of measuring pressure and/or force caused by external factors, and a second step that quantifies the amount and/or type of the drug to be applied based on levels identified in the first step. The first step can also include measurement of the elasticity of the muscles and/or eyelid distractibility and/or eyelid laxity, and applying medication in accordance with the levels identified.

The aspect of the invention related to the use of neurotoxins features the step of intramuscular administration (in the muscle) of a neurotoxin to a mammal, thereby alleviating the external pressure effects caused by muscle action and/or muscle tension. A preferred method for using neurotoxin includes the delivery of a concentration effective to reduce eye pressure caused by the eyelid muscle activity, blinking, or squeezing the eyes thereby reducing damage to the optic nerve.

According to the invention, the neurotoxin used is a botulinum toxin, such as one of, or a combination of one or more, of the botulinum toxin serotypes A, B, C, D, E, F and G. In addition, botulinum toxin can modulate the effects of glutamate, thereby reducing the glutamate-induced excitotoxicity which in association with increased eye
pressure can further damage retinal ganglion cells and the optic nerve. The neurotoxin can be used also in combination with other agents that increase its therapeutic effect such as calcium channel blocker (e.g.: verapamil), anesthesics (e,g,.: bupivicaine), and the like.

The invention features the administration of compounds or combination of compounds that modulate eye and facial muscle activity which can be applied invasively (percutaneous, intramuscular). In particular, the invention features use of compositions such as the combination of: lidocaine and alcohol (e.g.: ethanol). This combination of compounds acts primarily on muscle spindle afferent activity. Other useful compounds not according to the invention include doxorubicin, gabapentin, phenol, anticholinergic drugs, tetrabenazine, lisuride, lidocaine, mexiletine, trihexyphenidyl, and the like.

It is an object of the present invention to provide new methods and drugs for the treatment of various eye disorders by acting on the eyelid muscles and facial musculature and by modulating muscle tension, blinking, closure of the eye, squeezing of the eyelids, and the like. According to the invention, the drug is applied to the site of action by injection.

It is another object of the invention to administer muscle relaxants that are capable of reducing the effect of muscle contraction and blinking of humans or animals suffering from glaucoma or other conditions that can benefit from reducing eye pressure.

It is also an object of the invention to provide a method for maximizing the health of the optic nerve and retina by increasing retinal blood flow and increasing optic nerve head blood flow which comprises applying to the eye an effective amount of an agent that acts on at least one of the ocular muscle, eyelid muscle and facial musculature to promote a decrease in eye pressure, reduction of pressure spikes or reduction of pressure fluctuations.

It is still another object of the invention to provide a device for administering a drug such as a neurotoxin via iontophoresis besides piercing means. The device comprises means to apply electric current, a holding means to hold a drug, an activating means to activate the current so that it releases the drug, wherein said means is in contact with the skin.

It is also an object of the invention to provide novel methods and location for administering chemomodulating agents in a controlled and reproducible manner so as to confine their effects to a given region of muscle mass that can reduce eye pressure spike and fluctuation such as injecting the compound in the Riolan muscle of the lid margin.

It is yet an object of the invention to provide novel methods for treating facial and periocular wrinkles using a safe, painless and low cost compound.

An additional object of the invention herein disclosed is to provide a method for controlling eye pressure and neurotoxicity in glaucoma, ocular hypertension, optic neuropathy, optic neuritis, diabetic retinopathy, and the like using a neuro protective and lowering pressure agent, such as Botulinum toxin, that can be applied a few times a year instead of the conventional daily dose regimen for oral medications or eye drops.

It is yet another object of the invention to provide pharmaceutically effective amounts of a chemomodulating agent such as botulinum toxin alone or in conjunction with another drug. It is also an object of the invention to provide long lasting therapeutic approach to any situation in which there is a risk for increased eye pressure or risk of neuro toxicity caused by drugs (e.g: ethambutol) or neural damage by diseases such as mediated by glutamate and NMDA (N-Methyl-D-aspartate) receptors.

Another object is to provide novel dosage forms of such agents. Yet another object is to provide novel therapies for glaucoma and ocular hypertension heretofore untreatable or treatable only imperfectly with eye drops and other means.

A further object is provide a new vial with a particular amount of toxin. It is also an object to provide a new seringe for delivering the toxin. Yet another object is to provide a kit containing a vial and a new seringe and/or a Lid Measuring Device.

The invention features the use of the above named compounds for treating glaucoma.

An enhancement of surgical outcome by modulating eyelid muscle tension, blinking and squeezing of eyelid prior, during, and after surgical procedures such as refractive surgery and cataract surgery is disclosed. Administering botulinum toxin as a neuroprotective agent that protects the nerve by working preferably as an antagonist of glutamate is disclosed.

The compounds of the present invention act in normal muscle to bring those muscles to a lower than normal strength in order to decrease the eye pressure effect caused by said muscles. The methods disclosed herein target muscle that are responsible for the increased eye pressure while preserving the function of the other lid muscles responsible for lid functioning. The compounds of the present invention preferably act to reduce or eliminate the increase in pressure caused by muscle action or tension, and to decrease the velocity of the down phase of blinking.

The methods disclosed herein include using an amide-based anesthetic prior to the injection of botulinum toxin or lidocaine-alcohol composition, or prior to the use of doxorubicin, or any other agent that acts as a muscle relaxant. The injection of bupivicaine can lead to an amplification of effect of muscle relaxants. Studies by the present invention showed that pretreatment with local anesthetic helps to optimize the chemomyectomy effect.

Clinical studies demonstrated that the clinically used concentrations and combinations of the compounds disclosed herein cause a modulation of muscle action and EPE and provide a therapeutic effect for treating various conditions. An exemplary clinical study will be described.

### A. The Inclusion Criteria for the study included:

a. Patients with glaucoma who progressed despite normal or low IOP.
b. Patients who at the time of diagnosis had advanced damage to optic nerve head (ONH)/visual field (VF).
c. Patients who want to postpone or avoid glaucoma surgery.
d. Patients who want to postpone adding a new eye drop to the regimen.
e. Patients who want to try a non-eye drop based IOP lowering drug.
f. Patients who had a noticeable or measurable eyelid effect.
g. Patients who had allergy to multiple eye drops

All patients had to agree with off-label use of drugs to treat eye disorders.

### B. The Purpose of the,study included:

1. Evaluate the reduction of pressure spikes, pressure fluctuation, and baseline IOP
   caused by external pressure factors
2. Evaluation of dosages and compositions that keep pressure spikes and pressure fluctuation caused by external factors below Target Pressure
3. Evaluation of dosages and compositions that increase and stabilize ocular perfusion pressure by reducing external pressure effects
4. Evaluate various drugs that reduce the external pressure effects
5. Evaluate force and speed of muscle activity
6. Evaluate compounds that weaken the force of muscle groups which cause increase in eye pressure
7. Evaluate compounds that reduce velocity of phase-down eyelid motion
8. Evaluate synergistic effect between drugs reducing EPE and eye drops to treat glaucoma such as prostaglanding analogues
9. Quantify the magnitude of pressure spikes, pressure fluctuation, and baseline IOP caused by external pressure factors pre- and post-therapy
10. Quantify force and speed of muscle activity pre-and post-therapy
   a) Velocity of phase-down eyelid closure
   b) Force of muscle groups causing increase in eye pressure
11. Evaluate ocular blood flow during action by external pressure effects, (e.g., blinking and squeezing the eyes) pre- and post-therapy
12. Evaluate EPE in normal individuals and patients with eye disorders

### C. Methods of study:

Average age of treated patients was 61.9 years of age and 32 patients were treated over time, but criteria of a minimum of 8 months of consistent follow-up was applied, reducing the sample population to 10 patients. There were 8 Male and two Female. The Mean IOP pre-therapy: 24.9 ± 4.6. Eye drops pre-study therapy according to number of patients were as following: two patients were using prostaglandin analogue (PGA), betablocker (BB), alpha agonist (AA), and carbonic anhydrase inhibitor (CAI). Two other patients were using PGA, BB, and AA. Two other patients were using PGA alone. Two patients were using PGA, BB and CAI. One patient was using BB and CAI, and one patient who was eye drop intolerant was using BB alone.

All patients had IOP measured using Goldmann tonometer at the same time period. Changes in blood flow due to EPE was evaluated using color Doppler imaging and Blue Field Entoptic Phenomena. Each patient was treated and followed individually, not as a group. The study concerns data collected over time. Treatment interval for each patient varied due to logistical situations, an average of three months for botulinum toxin (seven patients) and six weeks for lidocaine composition (three patients). External eye pressure effects during blinking and squeezing were measured using a specially designed pressure sensing contact lens-assembly based on the device by Abreu disclosed in U.S. Patent No. 6,120,460. A baseline of the pressure effect of the lid muscle activity is then obtained. This gives information of how much eye pressure increase or eye pressure spike occurs due to muscle activity. Subsequent to that, lowering of eye pressure is accomplished by weakening muscles with percutaneous injections of the drugs adjacent to Riolan's muscle. Blockade of Riolan's reduce the increase eye pressure effect while maintaining normal eyelid function.

The two preferred compositions for lowering eye pressure used in the study were:

### 1. LIDOCAINE ALCOHOL COMPOSITION:

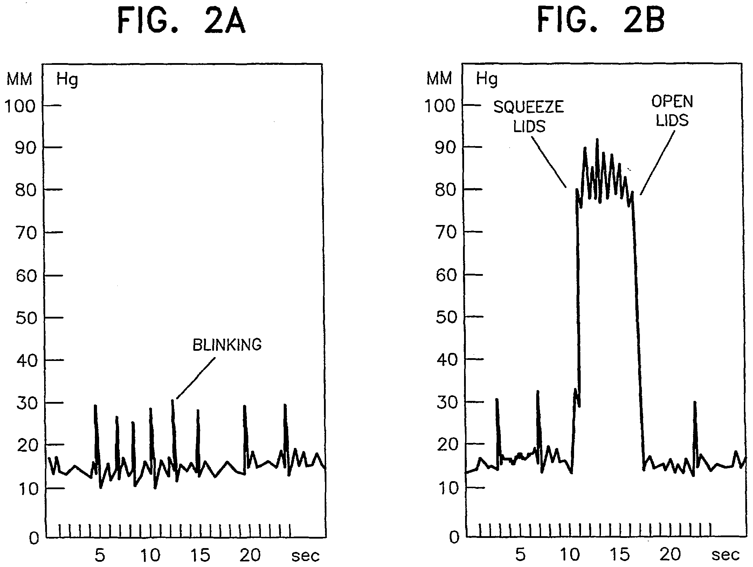

Injections comprised of lidocaine 1% added to 99.5% ethanol in one tenth of the volume of lidocaine. This composition affects muscle spindle afferent activity. A 27 gauge insulin syringe was used to perform the injections with glaucoma patients lying on an examination table or reclining chair with the eyes closed. 0.1 to 0.3 ml of this solution was injected into each site at the pretarsal orbicularis muscle at the lid margin (at sites (a) and (b) as shown in FIG. 2) adjacent to the muscle of Riolam and in the orbicularis muscle. Adjacent sites were used according to the clinical needs of the patients.

### 2.BOTULINUM TOXIN:

A dosage of 0.625 U up to 1.25 U of botulinum toxin type A was injected at the pretarsal orbicularis muscle at the lid at sites (a) and (b) adjacent to the muscle of Riolam and in the orbicularis muscle. A 27 gauge insulin syringe was used to perform the injections with glaucoma patients lying on an examination table or reclining chair with the eyes closed. Sites used were the same as for the lidocaine composition.

### D. Results (pressure are noted in mm Hg):

D1. Lidocaine Alcohol Composition: three patients received lidocaine alcohol composition:
   1. Patient 1: male, age 60 on four eye drops (PGA, BB, CAI, AA), IOP pre-therapy: right eye (OD) = 23, left eye (OS) = 20
      a. Criteria for inclusion: no IOP control and visual field (VF) progression OD
      b. Therapy (Rx) #1: Received four series of injections OD, interval of six to eight weeks per Rx
      c. IOP post Rx = 23, 18, 18 and 19 (% reduction of IOP for OD: 18%)
      d. Outcome: no VF progression
   2. Patient 2: male, age 39 on 1 eye drop (PGA). IOP pre Rx : OD = 24, OS = 27
      a. Criteria: pigmentary glaucoma and advanced VF defects
      b. Rx #1: Received one injection OS, then three series of injections both eyes (OU), interval of eight weeks per Rx.
      c. IOP post Rx: OD/OS = 25/23 - 24/23 - 22,22 and 20,22 (% of additional reduction of IOP: 16.7% for OD and 18.5% for OS)
      d. Outcome: no VF progression
   3. Patient 3: male, age 67, diabetic on 3 drops (PGA, BB, CAI). IOP pre Rx: OD = 28, OS = 26
      a. Criteria: moderate VF defects with progression and patient wants to postpone adding other eye drops to regimen
      b. Rx #1: Received six series of injections OU, interval of six to eight weeks per Rx
      c. IOP post Rx: OD/OS = 27,26 - 25,26 - 24,25 - 24,25 - 25,24 - 25,25 - 25-26 (% additional IOP reduction: 11% for OD and 7.6% for OS)
      d. Outcome: no VF progression - patient kept on three eye drops and no need to change regiment.
D2. Botulinum Toxin: seven patients received botulinum toxin
   1. Patient 1: male, age 60 on 4 eye drops (PGA, BB, CAI, AA). IOP pre Rx: OD = 18, OS = 17
      a. Criteria: no IOP control and VF progression OD, and patient wants to postpone surgery
      b. Rx #1: Received three series of injections OD, interval of three months per Rx
      c. IOP post Rx = 16, 15, and 15 (% IOP reduction for OD: 16.6%)
      d. Outcome: no VF progression - surgery postponed
   2. Patient 2: male, age 61 on 3 eye drops (PGA, BB, CAI) IOP pre Rx = OD 19, OS = 21
      a. Criteria: advanced VF defects with progression and patient cannot put eye drops on his own due to arthritis
      b. Rx #1: Received three series of injections OU, interval of about 2.5 months per Rx
      c. IOP post Rx = 17,18 - 20,17 and 17,18 (IOP reduction for OD was 10.5%/IOP reduction for OS was 14%)
      d. Outcome: no VF progression
   3. Patient 3: male, age 56 on 3 eye drops (PGA, AA, BB). IOP pre Rx: OD = 26, OS = 30
      a. Criteria: advanced VF defects and patient does not want eye drop-based therapy
      b. Rx #1: Received four series of injections OS only, interval of three to four months per Rx
      c. IOP post Rx: OD/OS = 26,29 - 25,31 - 30,32 and 29,30 (no reduction of IOP in the treated eye (OS) and 12% increase in IOP in the untreated eye)
      d. Outcome: no VF progression for OS and VF progression for OD
   4. Patient 4: female, age 63 on 3 eye drops (PGA, AA, BB). IOP pre Rx: OD = 20, OS = 21
      a. Criteria: advanced VF defects and continued progression and patient intolerant to CAI.
      b1. Rx #1: Received three series of injections OU, interval six months between first and second, then three month intervals
      c1. IOP post Rx: OD/OS = 18,18 - 24,22 and 19,20 (% reduction IOP was 12.5% OD and 10% OS in the 2 last visits)
      d1. Outcome: no VF progression
      Rx #2: Patient became intolerant to AA and received also lidocaine compostion.
      b2. Received three series of injections lidocaine alcohol composition OU, interval of two months per Rx
      c2. IOP post Rx on 2 eye drops only (PGA, BB): OD/OS = 16,18 - 18,18 and 18,19 (% reduction on 2 eye drops: 10% for OD and 9.5% for OS)
      d2. Outcome: no VF progression during treatment time
   5. Patient 5: male, age 59, diabetic on 1 eye drop (BB). IOP pre Rx: OD = 32, OS = 29
      a. Criteria: advanced VF defects and allergy to multiple eye drops
      b. Rx #1: Received two series of injections OD, interval of three months per Rx
      c. IOP post Rx: OD/OS = 30,29 - 30,28.
      d. Outcome: no VF progression OD, VF progression OS. No progression of diabetic retinopathy in treated eye
         Rx #2: due to improvement patient accepted therapy for both eyes. Therefore in therapy #2 the patient received two series of injections in both eyes, interval of three months per Rx
      e. IOP post Rx: OD/OS = 30,29 and 29,27 (% reduction IOP was 10% OD and 7% OS)
      f. Outcome: no VF progression OU
   6. Patient 6: male, age 71 on 2 eye drops (BB, CAI). IOP pre Rx: OD = 28, OS = 28
      a. Criteria: no IOP control, continued serial VF progression, and lack of compliance
      b. Rx #1: Received two series of injections OU, interval of four months per Rx
      c. IOP post Rx: OD/OS = 26,27 and 31,30 (IOP increase: +10% for OD and +11% for OS).
      d. Outcome: two serial VF with no VF progression. With only two injections and short follow up there was no observed IOP reduction however there was no VF progression and no visual loss since the spikes of eye pressure and eye pressure fluctuation due to muscle activity were controlled.
   7. Patient 7: female, age 67 on 1 eye drop (PGA). IOP pre Rx: OD = 26 (only seeing eye), OS is blind with pressure of 38 (history of vein occlusion).
      a. Criteria: moderate to advanced VF defects, continued progression, only eye and intolerant to eye drops.
      b. Rx #1: Received nine series of injections OD of both botulinum toxin (5) and lido composition (4), interval of two to eight months per Rx.
      c. IOP post Rx during first 3 Rx with botulinum toxin: 26 - 24 - 24 - 23 (% IOP reduction for OD was 11.5%)
      d. Outcome: minimal VF progression
         Rx #2: patient received 2 series of botulinum toxin and 4 series of lidocaine alcohol composition
      e. IOP post Rx: OD = 25, 24, 25, 23, 23 and 21 (% IOP reduction for OD was 19.2%)
      f. Outcome: no VF progression

It was observed that a significant enhancement of IOP lowering therapeutic effect of PGA when used in association with the compounds in accordance with the present invention. Blood flow was reduced during EPE and enhanced after treatment with the compounds disclosed herein. No patient complained about side effects related to ocular surface integrity nor changes in the surface of the eye were observed during physical examination. Further detailed description of experiments and findings are disclosed in the Detailed Description section.

The various lines of clinical evidence presented in the present invention show the following:
1. Reduction of external pressure effects (EPE) seemed to lead to a fairly uniform preservation of visual field.
2. Eyes not treated seemed to progress.
3. Treatment seemed to consistently decrease baseline IOP.
4. Treatment with the compositions was the most important factor for preventing VF progression despite minimal baseline IOP lowering in some patients. Reason for that is possibly due to three factors:
   a. Reduction of times in which eye pressure goes above target pressure;
   b. Reduction in the number of pressure spikes and pressure fluctuation; and
   c. Increase in ocular perfusion pressure and blood flow by modulating increased resistance caused by consistent pressure spikes and pressure fluctuation.
5. Reduction of pressure spikes and pressure fluctuation by reducing the phase-down velocity of eyelid motion
6. Obtaining a synergistic effect with IOP lowering eye drops and enhancing the IOP lowering response of eye drops, particularly PGA. This may be due to:
   a. Increasing physical stability of the eye which may optimize the effect of eye drops;
   b. Direct effect of therapy by reducing action by muscle and/or tarsal plate against the cornea; and
   c. Combination of effects.
7. Lowering eye pressure, eye pressure spikes, and pressure fluctuation and preventing visual filed progression while preserving normal eyelid function.
8. Increasing ocular blood flow, and thus be beneficial in hypoxic states such as diabetic retinopathy, retinal vein occlusion, ischemic optic neuropathy, and the like.
9. Reducing or maintaining the number of eye drops if pressure fluctuation and pressure spikes due to EPE are adequately controlled.
10. The clinical studies indicated that the large and frequent increase in eye pressure caused by muscle action can lead to eye damage and treatment in accordance with the present invention can prevent said eye damage. Other therapeutic clinical benefits included:
   a. Vision was preserved in treated eyes;
      a. Reduction of IOP, pressure spike and pressure fluctuation;
      b. Treated eye remained stable and untreated lost VF;
      c. There was reduction in the number of eye drops used for the treated eye;
      d. There was enhancement of effect of eye drops used;
      e. There was prevention of visual field progression in the treated eye; and
      f. Surgery was postponed for the treated eye.

Other results, features, and advantages of the invention will be apparent from the detailed description, accompanying drawings, and the claims.

### BRIEF DESCRIPTION OF THE DRAWINGS

FIG. 1 is a cross sectional schematic view showing the eyelid pressing against the eye.
FIG. 2A is a graph showing the increase in eye pressure caused by blinking.
FIG. 2B is a graph showing the increase in eye pressure caused by squeezing the eyelids.
FIG. 3 is schematic view showing areas for administering the compounds of the present invention.
FIG. 4A is a graph showing the EPE due to blinking prior to administration of a lidocaine composition.
FIG. 4B is a graph showing the EPE due to blinking after administration of a lidocaine composition.
FIG. 5A is a graph showing the EPE due to squeezing prior to administration of a lidocaine composition.
FIG. 5B is a graph showing the EPE due to squeezing after administration of a lidocaine composition.
FIG. 6A is a graph showing the EPE due to blinking prior to administration of botulinum toxin.
FIG. 6B is a graph showing the EPE due to blinking after administration of botulinum toxin.
FIG. 7A is a graph showing the EPE due to squeezing prior to administration of botulinum toxin.
FIG. 7B is a graph showing the EPE due to squeezing after administration of botulinum toxin.
FIG. 8A is a schematic diagram showing a kit that can be used for the practice of the invention.
FIG 8B is a schematic view showing a new device that can be used to practice the invention.
FIGS. 9A and 9B are schematic diagrams showing exemplary Lid Measuring Devices (LMD).

### DETAILED DESCRIPTION OF THE PREFERRED EMBODIMENTS

In describing a preferred embodiment of the invention illustrated in the drawings, specific terminology will be resorted to for the sake of clarity. However, the invention is not intended to be limited to the specific terms so selected, and it is to be understood that each specific term includes all technical equivalents which operate in a similar manner to accomplish a similar purpose.

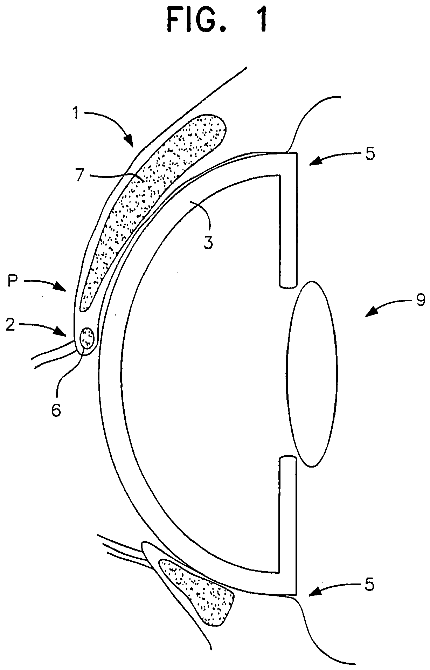

FIG. 1 is a cross sectional schematic view showing the eyelid 1 causing an increase in eye pressure due to the eyelid margin 2 and Riolan muscle 6 as said eyelid 1 descends during blinking and covers the cornea 3. As the upper eyelid 1 descends during blinking, said eyelid 1 pushes down and squeezes the cornea 3 causing an increase in eye pressure.

In individuals with normal blinking function, involuntary blinking occurs approximately every 2 to 10 seconds creating an almost continuous increase in eye pressure which happens each time one blinks. In normal individuals the fluctuation of pressure with blinking can reach 10 mm Hg, but with glaucoma patients blinking can increase IOP over 25 mmHg. When squeezing the eye the eyelid 1 can generate an increase of over 90 mm Hg, with said enormous pressure elevation being short lived in normal people but sustained in glaucoma patients. During sleep since there is non-interrupted pressure by the eyelid 1, besides rapid eye movements, and involuntary pressing of the eye against physical objects, the increase in eye pressure can lead to further damage.

Three physical factors were identified as playing a role in EPE and said EPE potentially blinding effects, and include Eyelid Motion, Vector Forces in the Eyelid, and Phases of Blinking and Eyelid Velocity. The physical factors were also carefully analyzed and are described in detail below.

### 1. Eyelid Motion

During its closing phase the upper eyelid 1 descends from the open position until it meets the upper part of the cornea 3. The cornea 3 is then pushed inward with the eyelid margin 2 making a re-entrant angle of about 35 degrees with the cornea 3. After making this initial small excursion the eyelid 1 then slides over the outer surface of the cornea 3 squeezing the cornea 3 and covering said cornea 3. During blinking when the eyelid 1 is in contact with the cornea 3 there is an inward motion of the cornea 3 with significant increase of the eye pressure in glaucoma patients. The impact caused by the inward motion and squeezing of the eye starts at the corneo-scleral junction 5 due to a slope change of about 13 degrees in the area of intersection between cornea 3 (radius of 9 mm) and sclera 4 (radius 11.5 mm).

### 2. Vector Forces in the Eyelid

A combination of vector forces caused by the contraction of the muscle of Riolan 6 near the rim of the lid 2 and orbicularis muscle 7 are applied to the cornea 3 by the eyelid 1. A horizontal force (normal force component) of about 20,000 to 25,000 dynes and a vertical force (tangential force component) of about 40 to 50 dynes is applied on the cornea 3 by the upper eye lid 1, which can create a significant increase in the eye pressure of glaucoma patients. The force applied by muscle action on the eye is so significant to the point of moving the whole eye ball 9 inward from 1 to 6 mm as the upper eyelid 1 descends during the closing phase of the blink. The force applied by the eyelid 1 at rest on the cornea 3 also causes a significant increase of the baseline eye pressure in glaucoma patients that can be alleviated in accordance to the present invention.

### 3. Phases of Blinking and Eyelid Velocity

Blinks normally consists of a fast (up to 2,000 degrees/sec), large downward eyelid movement lasting about 50 msec followed by a slower up phase. The descent of the upper eyelid 1 reaches its maximum speed at about the time that it crosses the visual axis, generally in levels of 20 cm/sec. Sometimes with forceful blink the speed can reach over 35 cm/sec, or even more. This phase-down velocity of blinking and, during closing the eye is a key component of the increased eye pressure due to external pressure effects.

Physically, a particle of mass "m" subjected to force "F" and moving with velocity "v" posses a kinetic energy. Since P=F/a, by decreasing velocity, the force impacting the cornea is reduced, with the subsequent reduction of pressure effect, whrein P is pressure, F is Force, and a is area.

The present invention provides means to control and reduce EPE by slowing blink peak velocity and force of the blink-down phase. The invention also includes drugs (neurotoxin and anesthetic compositions) to pharmacologically achieve the control of
eye pressure and lowering of eye pressure caused by the external pressure effects while preserving adequate blinking and eyelid function.

FIG. 2A is a graph showing the increase in eye pressure caused by blinking. Each spike of IOP corresponds to blinking. Every time a person blinks there is an increase in eye pressure, varying from 5 to 10 mm Hg. However in glaucoma patients the increase can reach 25 mmHg. This continuous and 16,000 times daily average pressure spike can lead to damage to the eye over time since the drainage system in glaucoma is not working well, preventing fluid from leaving the eye and therefore preventing the eye pressure to be normalized in a timely manner.

FIG. 2B is a graph showing the increase in eye pressure caused by squeezing the eyelids. The IOP rapidly increases to very high levels when there is squeezing of the eyelids and return to its baseline or close to its baseline when the eyelids are opened, as indicated in the figure. Every time people squeeze their eyes there is a tremendous increase in eye pressure, reaching even 100 mg Hg (normal eye pressure is bellow·21 mm Hg). In glaucoma patients there is a higher increase of eye pressure which is sustained and return to normal levels can take a longer time depending on the functioning state of the drainage system of the eye. The exceedingly high IOP can reduce perfusion pressure and cause direct damage to the optic nerve.

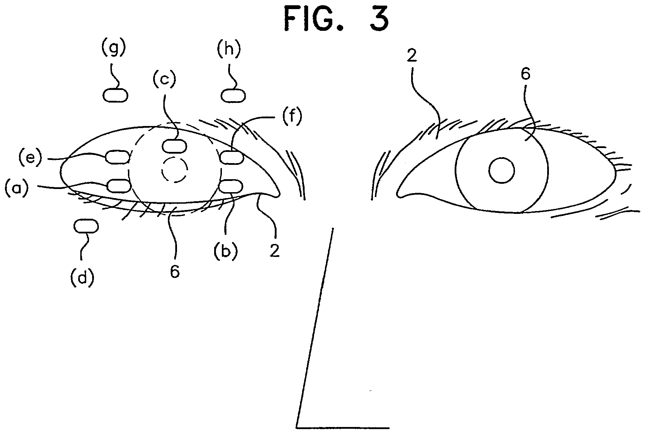

FIG. 3 is schematic view showing areas for administering the compounds of the present invention including areas of injection for the toxin, sites (a) and (b). Sites (a) and (b) are in the eyelid margin 2 adjacent to the muscle of Riolan 6, and site (c) is located in the orbicularis muscle of the upper eyelid 1. Site (d) is located in the lateral aspect of inferior eyelid. Sites (e) and (f) are located in the upper eyelid 1 and sites (g) and (h) are located above the eyebrow. The right eye is closed and the left eye is open for illustrative purposes.

Generally, for bilateral glaucoma, a starting dose of the compounds disclosed herein is applied at only two sites (sites a and b) adjacent to Riolan's muscle 6. Alternatively, the compound is injected at three sites of each eye, using sites (a), (b), and (c).

In addition, in more difficult to control cases, one site in the orbicularis muscle of the lower eyelid (site d) can be used for a total of four sites. For more advanced glaucoma, five sites in each eye can be injected, and using then sites (e) and (f), in addition to site (a), (b), and (d). More injections sites in the eyelid, face, and above the brow (sites c, g, and h) are used when significant muscle activity is present and/or if the glaucoma is advanced and the patient is at risk of going blind in a short time due to pressure spikes. Higher dosages of the compound per site can be used for more advanced glaucoma. Thus the number of sites and the amount of toxin can vary according to the clinical response and the stage of the disease.

With an appropriate dose, because the muscle is only partially and superficially weakened, enough strength and neural control remain so that a treated muscle still can perform its primary voluntary function and the patient can blink normally but with less force and at a lower speed. One of the ways to optimize the therapeutic effect is by creating a Pharmacological Speed Bump. The phase-down velocity is then reduced from 28 cm/sec to 16 cm/sec after the therapy (velocity in physics is defined as speed and direction of motion). The key is to create a Pharmacological Speed Bump in which a drug acts by decreasing the speed of phase-down without affecting frequency of blinking. Riolan's muscle is the accelerator that needs to be modulated by the agent, thus blockade of Riolan 's muscle reduces the velocity. The degree of weakening from modulation can be titrated empirically for particular patients by altering the dose according to the status of their optic nerve.

Under physiological conditions, contractions in muscular tissues is the end result of a sequence of processes which begins with neural activation and the generation of electrical signals in the cellular surface membrane, influx or release from stores of calcium ions, activation and cross-bridge formation between actin and myosin filaments. Any compound acting on any of those stages can be used according to the principles of the invention.

The present description includes a method of preventing neural toxicity and lowering eye pressure by modulating the function of eyelid muscles and facial muscles through acting in any of the aforementioned stages of muscle contraction.

Accordingly, one embodiment includes a composition acting by promoting neural blockade and by reducing muscle spindle activity and cross bridge formation. This embodiment uses low cost compounds which facilitates the universal use of the therapy.

This embodiment includes the use of a combination of lidocaine as local anesthetic drug combined with alcohol (ethanol and/or isopropyl alcohol). The local anesthetic drug blocks nerve impulses by interfering with the opening of voltage gated sodium channels of excitable membranes, such as neuronal cell membranes. The combination with alcohol creates a transient motor blockade and muscle relaxation due to neural blockade and reduction of muscle spindle afferent activity. The therapeutic effect is concentration-dependent, thus a higher concentration is used for a higher degree of blockade.
The compound to be combined with alcohol is lidocaine, exemplary lidocaine 0.5% to 5% (Diethylaminoacet-2,6-xylidide).

This preferred embodiment includes the use of a combination of compounds, preferably lidocaine 0.5% to 1.0% (tradename "XYLOCAINE", from AstraZeneca, United Kingdom) added to 99.5% ethanol in one tenth of the volume of lidocaine. However, the total content of ethanol and/or isopropyl alcohol and compound varies depending on, for example, the types of compounds and other active ingredients present. An alternative embodiment n o t according to the invention includes the use of bupivicaine 0.5% added to 100% ethanol in one tenth of the volume of bupivicaine. In addition, 30 micrograms of can be added to the bupivicaine-ethanol clonidine composition in order to prolong the therapeutic effect. This composition reduces neural activity and muscle spindle afferent activity and promotes muscle relaxation. The duration of the effect can be short or long up to six weeks depending on the concentration and compounds used. 0.2 ml of this solution injected into the pretarsal orbicularis muscle has a similar effect, but of lesser duration compared to using botulinum toxin. The solution is preferably injected in the medial and lateral parts of the edge of the upper eyelid in a similar manner as for the neurotoxin.
An example of using muscle spindle afferent receptors as the target to treat glaucoma include the following. A 60 year old glaucoma patient reclined on a standard examining chair and with the eyes closed was injected at sites (a) and (b) shown in FIG. 3 with a composition comprised of 2 ml of lidocaine 0.5% added to 99.5% ethanol in one tenth of the volume of lidocaine.A 30 gauge insulin syringe was used to perform percutaneous injections of 0.2 to 0.3 ml of the composition into the pretarsal orbicularis muscle at the lid margin adjacent to the muscle of Riolan. Percutaneous herein refers to any technique piercing the skin to deliver the compound. The weakening of the muscles results in the decrease of: eye pressure spikes, baseline eye pressure, and eye pressure fluctuations. There was 26 to 30% decrease in eye pressure after forceful closure of the eyes and during blinking, besides a three week average 16% reduction of baseline eye pressure. The pharmacological blockade as performed did not lead to any disturbance of lid function and there were no subjective complaints by the patient.

Another preferred embodiment includes a neuromuscular blocking agent such as an acetylcholine transmission inhibitor for percutaneous injection using, for example, a 27 to 30 gauge needle in a 1 ml tuberculin syringe, and preferably using a newly designed 0.5 ml syringe. The drug is used for injection into the musculature at the eyelid margin adjacent to the muscle of Riolan which lead to weakening of the muscles which in turn results in the decrease of: eye pressure spikes, baseline eye pressure and eye pressure fluctuations. The compounds disclosed herein enable maintenance of this modulating state with a single injection lasting several months.

The invention includes botulinum toxin for injection.. Other toxins which are no compound according to the invention, but useful include tetanus toxin, tetrodotoxin, spider venoms and various animal venoms. Proteins produced using recombinant DNA technology which mimic the effects of these natural materials can also be used. Examples of drugs that are no compound according to the invention and can be used by mouth include Baclofen, benzotropine mesylate, clonazepan, gabapentin, and the like.

The preferred neurotoxin of the invention is botulinum toxin serotype A which is a two-chain protein with an average molecular weight of about 140 to 150 thousand daltons. The toxin acts at the neuromuscular junctions to inhibit acetylcholine release from the presynaptic membranes and produces a dose-related weakness or loss of muscle tone or partial paralysis of muscles. The heavy chain of the toxin binds to the specific receptor of cholinerigc nerve endings, and the toxin acts by inhibiting acetylcholine release from presynaptic ending at the neuromuscular junction.

Botulinum toxin blocks transmission of nerve impulses acting as a muscle relaxant and temporarily relaxing certain eye muscles leading to decreased eye pressure and decreased eye pressure spikes. Serotype A is commercially available from Allergan, Inc. of Irvine, California under the tradename "BOTOX" and by Porton Products Ltd., of the United Kingdom under the tradename "DYSPORT". A pentavalent toxoid of all eight known Botulinum serotypes is also available as an investigational drug from the U.S. Center for Disease Control in Atlanta, GA, and from the Eye Research Foundation, San Francisco, California.

Another preferred neurotoxin is botulinum toxin serotype B, commercially available from Elan Corporation of Ireland, under the tradename "MYOBLOC". Serotypes B, C and F are also available from WAKO Chemicals of Japan. Tetanus toxins for use as vaccines can also be used according to the present invention and are commercially available from Lederle Laboratories of Wayne, N.J. under the tradename "TETANUS TOXOID PUROGENATED". The Ibc fragment of the tetanus toxin is believed to act peripherally and is therefore similar in its activity to Botulinum toxin.

Both botulinum toxin and tetanus toxin are zinc endopeptidases that enter nerve cells and block neurotransmitter release by impairing the fusion of vesicles with the presynaptic membrane. On a cellular level, the mechanism of action of clostridial neurotoxins occurs in three steps: cell binding to a surface receptor, internalization and finally intracellular poisoning that inhibits acetylcholine release. The heavy chain accounts for the binding and internalization steps, and the light chain interferes with a zinc-dependent cleavage necessary for neurotransmitter release

The only commercially available ready-to-use solution is serotype B. Serotype A comes as powder to be reconstituted with fluid by adding a diluent such as normal saline 0.9% without a preservative. Toxins may be compounded into a pharmaceutical preparation, using pharmaceutical compounds well-known in the art; the exact formulation and dosage of the compound depends upon the condition being treated and status of disease. Generally, the effective dose of the toxin for treating glaucoma and ocular hypertension and for increasing perfusion pressure is preferably between 0.025 and 0.05 ml at each site (considering a vial with 100 U of dried toxin reconstituted with 8.0 ml of diluent). It is understood that an amount of 0.025 ml may be used in cases of mild spikes and/or mild glaucoma and dosages of 0.1 ml or more can be used in advanced cases. The technique includes applying the most effective dose in the smallest possible volume of liquid. Other dosages can be used according to the clinical needs of the patient. Subsequent doses may be increased to twice or three times the original amount, however the guidance for the amount of dose is preferably done clinically by observing the reduction of the spike in eye pressure according to methodology described below using Lid Measuring Devices.

For bilateral glaucoma in general, and referring to FIG. 3 for site location; a starting dose of 2 U of botulinum toxin type A is preferably applied at only two sites (sites a and b) adjacent to Riolan's muscle 6, at a dosage of 1 U of botulinum toxin A per site. Alternatively, 1.875 U of botulinum toxin A (Botox) is injected at 3 sites of each eye, at a dosage of 0.625 per site, using sites (a), (b), and (c).

In addition, in more difficult to' control cases, one site in the orbicularis muscle of the lower eyelid (site d) can be used for a total of four sites and injection totaling 2.5 U. For more advanced glaucoma, five sites in each eye can be injected for a total of about 3.75 U of toxin by using sites (e) and (f), in addition to sites (a), (b), and (d). More injections site in the eyelid, face, and above the brow (sites c, g, and h) are used when significant muscle activity is present and/or if the glaucoma is advanced and the patient is at risk of going blind in a short time due to pressure spikes. Higher dosages ranging from 1U to 5U per site can be used for more advanced glaucoma. Thus the number of sites and the amount of toxin can vary according to the clinical response and the stage of the disease.

The purpose of the injections is to reduce the speed and/or force of: the eyelid muscles, blinking and the forceful closure of the eyelids, for example for a period of months. This is accomplished by weakening muscles with injections, located preferably at the lid margin adjacent to the muscle of Riolan and in the orbicularis muscle, at a dosage of about 0.05 ml to 0.1 ml at each site - equivalent of 0.625 U and 1.25 U of Botulinum toxin type A respectively, considering a vial with 100 U of Botox reconstituted with 8.0 ml of diluent such as 0.9% sodium chloride. The degree of blockage of muscle activity can be regulated by variation of dosage, variation in the site of injection and frequency of injection. Higher dosages or lower dosages can be employed provided the dose is effective in reducing muscle activity causing EPE while being non-toxic. The effects of the toxin generally last for a defined period of time up to many months, but varies among patients. Injections may be repeated as needed to control the disease and the eye pressure.

The preferred method comprises the weakening of a particular muscle or group of muscles by local application of a compound. It is understood however, that weakening of the muscles can also be achieved by compounds acting in the central nervous system or peripheral nerves. The blink reflex is mediated by trigeminal nerve afferents and facial nerve efferents. There is an early ipsilateral response (R1), which is transmitted through an oligosynaptic pontine pathway, and a late bilateral response (R2) relayed through a polysynaptic medullar arc. Any compound acting on this neural pathway can be used, however the preferred compounds have minimal to no effect on those neural pathways because it is important to keep the frequency of blinking unchanged for preservation of normal eyelid functioning.

The preferred method of the invention provides minimal to no effect on the frequency of muscle action while achieving modulation of the speed of eyelid and modulation of the force of the eyelid muscle. The preferred method comprises local administration of the compound. This local action of the compound in the eyelid or vicinity of the eyelid allows preservation of the frequency of blinking. The frequency of blinking is necessary for adequate wetting of the cornea and for preservation of tear film stability and preservation of ocular surface integrity. Because the frequency of blinking is determined by the central nervous system, by acting locally the frequency of blinking is maintained. By not interfering with the frequency of blinking, the normal eyelid function is preserved.

In addition, by blocking only the muscles that are responsible for the increase in eye pressure and keeping intact the remaining muscles, eyelid function is further preserved. The muscles necessary for initiating the closure of the eyelids in phase-down as well as the muscles necessary for phase-up of the blinking are intact. The muscles responsible for the pressure increase, however, are partially blocked in order to decrease the peak velocity of the down-phase of blinking, and to decrease the force during squeezing of the eyelids. The change in velocity and the change in force due to pharmacological modulation of muscle action is not perceptible with the patient being treated, nor perceptible to an external observer looking at the patient during blinking or forceful closure of the eyes. The compounds act preferably locally at the muscle, or in the neuro-muscular junction, or at the nerve.

Therapeutic approach in accordance with the present invention aims to reduce the effect of muscle action and/or muscle tension on the eye, which causes changes in intraocular pressure. Studies indicated that speed by which the lid hits the cornea is one of the main factors causing the external pressure effect. One of the novel ways by which the present invention provides a therapeutic effect is by creating a "Pharmacological Speed Bump". A "Pharmacological Speed Bump" means reducing the speed of the eyelid motion by pharmacologically blocking the muscles responsible for the velocity of the eyelid, thereby reducing the velocity by which the eyelid impacts the cornea. By reducing the impact of the eyelid on the cornea there is a reduction of the pressure effect. The preferred method, therefore, is by changing the velocity of the phase-down motion of the eyelid.

Blinks normally consists of a fast (up to 2,000 degrees/sec) large downward lid movement lasting about 25 to 50 msec followed by a slower up phase. The descent of the upper eyelid reaches its maximum speed at about the time that it crosses the visual axis, generally in the range of 17 to 20 cm/sec. Some times with forceful blink the speed can reach over 40 cm/sec. This phase-down velocity of blinking and during closing the eye is one of the key components of the increased eye pressure and EPE. The pressure applied by muscle action on the eye is so significant to the point of moving the whole globe posteriorly up to 6 mm. If the patient has glaucoma that is translated into an enormous amount of pressure inside the eye.

For the down phase of the blink, the peak velocity as well as its duration can be modulated with the therapeutic approach of the present invention. The initial down phase of blinking and closing the eye increased in latency and decreased in peak velocity. The down phase of a 15 degrees blink was three times slower after therapy in accordance with the principles of the present invention. The phase-down velocity of the eyelid has been reduced to about 25 cm/sec with the proposed preferred compounds and dosages compared to 40 cm/sec before administration of the compounds.

This reduction in phase-down velocity is not perceived by the patient or externally, but the physical factors creating increase in pressure are modulated with the consequent reduction in eye pressure. It was discovered that injection in the Riolan muscle at the doses described provided the needed therapeutic effect for the different compounds mentioned, which reduces the peak velocity of blink down-phase, with no change in the frequency of blink.

The impact and force of the tarsal plate applied to the cornea can also increase eye pressure. By blocking the muscle of Riolan there is also a decrease in the effect of the tarsal plate on the eye. The upper eyelid tarsal plate is a large plate of connective tissue inside the eyelid with mean diameter over the eye in medium position of 19.3 +/-3.8 mm and 30.1 +/- 6.3 mm in abduction position. The tarsal plates and tension at the Riolan muscle causes an increase in the baseline eye pressure of glaucoma patients. Other preferred means to prevent the increase in eye pressure act by a combination of decreasing the effects of the tarsal plate as well as decreasing peak velocity and impact of the lid upon the cornea. The discovery that blocking the eyelid muscle with the proposed compounds and doses optimizes the therapeutic effect for both muscle action and tarsal plate effect while maintaining normal lid function and allows the effective and safe treatment of glaucoma and any other disorder that can benefit from reduction of eye pressure.

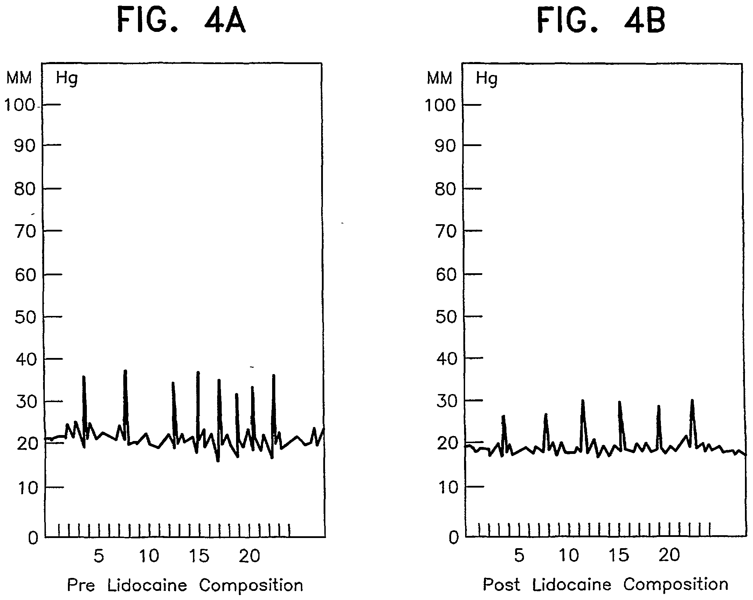

FIGS. 4A and 4B shows graphs demonstrating a decrease in the eye pressure during blinking in mm Hg after injection of the lidocaine and alcohol composition in the preferred manner described herein. X corresponds to time in seconds and Y corresponds to eye pressure in mm Hg. The graph in FIG 4A shows blink before administration of the lidocaine and alcohol composition (control). The graph in FIG.4B shows blinking after administration of the lidocaine and alcohol composition. Each spike of eye pressure corresponds to blinking. The higher spikes shown in the graph of FIG. 4A correspond to higher eye pressure by the blinking. The magnitude of pressure spike per blink in the graph of FIG. 4A (pre-injection) is greater than in the graph of FIG. 4B (post- injection). There is a decrease in eye pressure of 5 mm Hg per spike per blink which was considered as a positive effect. FIG. 4B also demonstrates the decrease in the force of muscle contractions of the eyelid (blinking) and decrease in speed of the phase-down velocity as revealed by the decrease in eye pressure per blink and slope of the spike after injection of lidocaine compared to control (control=pre injection of botulinum toxin). A reduction of baseline eye pressure was also noted.

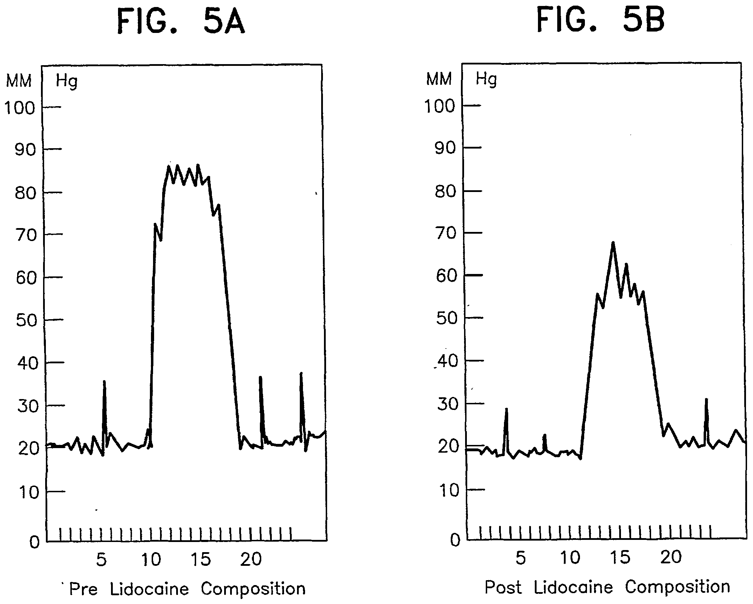

FIGS. 5A and 5B shows graphs demonstrating greater and sustained increase of eye pressure during forceful squeezing of the eyelids, which is reduced after administration of lidocaine composition. The increase in eye pressure was maintained until the lids were open. X corresponds to time in seconds and Y corresponds to eye pressure in mm Hg. The graph of FIG. 5A shows squeezing the eyelids before administration of lidocaine composition (control). The graph of FIG. 5B shows squeezing the eyelids after administration of lidocaine composition. The higher and sustained spike observed pre-injection shown in the graph of FIG. 5A corresponds to higher eye pressure by the forceful closure of the eyes. Although the ability to squeeze the eye and increase eye pressure is preserved after injection of the compound as shown in the graph of FIG. 5B, the magnitude of the pressure spike during forceful closure is reduced when compared to the magnitude of pressure elevation in the graph of FIG. 5A (pre-injection). It was noted that the forceful squeezing of the eyelids after receiving lidocaine composition leads to about 30% lower increase in the maximal eye pressure level (graph of FIG. 5B) when compared with the control as depicted in the graph of FIG. 5A.

An example of using the presynaptic membrane at the neuromuscular junction as the target to treat glaucoma in accordance with the principles of the invention include the following. A 65 years old glaucoma patient reclined on a standard examining chair and with the eyes closed was injected with botulinum toxin type A at sites (a) and (b) shown in FIG. 1. A 30 gauge insulin syringe was used to perform percutaneous injections with a dosage of 1.25 U at the lid margin adjacent to the muscle of Riolan and orbicularis muscle. There was an approximate 35% reduction in eye pressure after forceful closure of the eyes, besides a 12 week period average 16% reduction of baseline eye pressure. The pharmacological blockade as performed did not lead to any apparent disturbance of lid function and there were no subjective complaints by the patient.

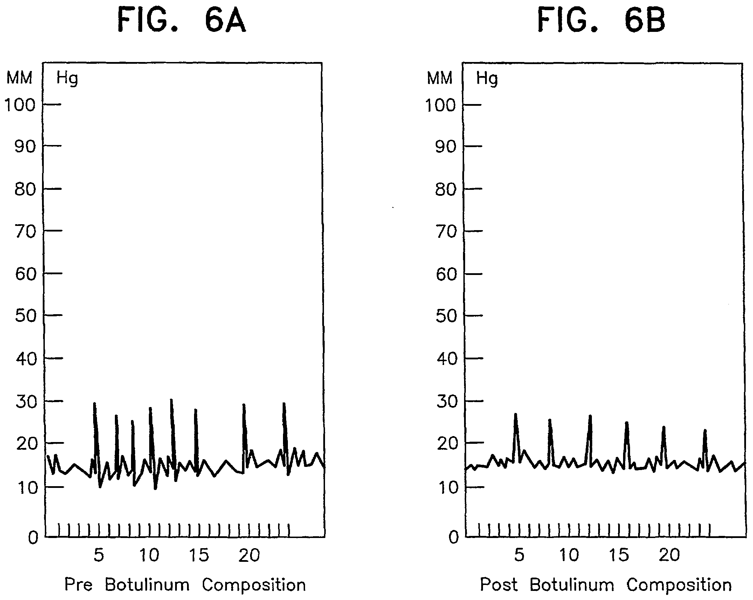

FIGS. 6A and 6B shows graphs demonstrating a decrease in the eye pressure during blinking in mm Hg after injection of Botulinum toxin in the preferred manner described herein. X corresponds to time in seconds and Y corresponds to eye pressure in mm Hg.

The graph in FIG. 6A shows a blink before administration of botulinum toxin (control). The graph in FIG.6B shows blinking after administration of botulinum toxin.. Each spike of eye pressure corresponds to blinking. The higher spikes observed pre-injection shown in the graph of FIG. 6A correspond to higher eye pressure by the blinking. The magnitude of pressure spike per blinking in the graph of FIG. 6A (pre-injection) is greater than in the graph of FIG. 6B (post- injection). There is a decrease in eye pressure of more than 5 mm Hg per spike per blinking which was considered as a positive effect. FIG. 6B also demonstrates the decrease in the force of muscle contractions of the eyelid (blinking) and decrease in speed of the phase-down velocity as revealed by decreased eye pressure per blink after injection of botulinum toxin compared to control (control=pre injection of botulinum toxin). A reduction of baseline eye pressure was also noted.

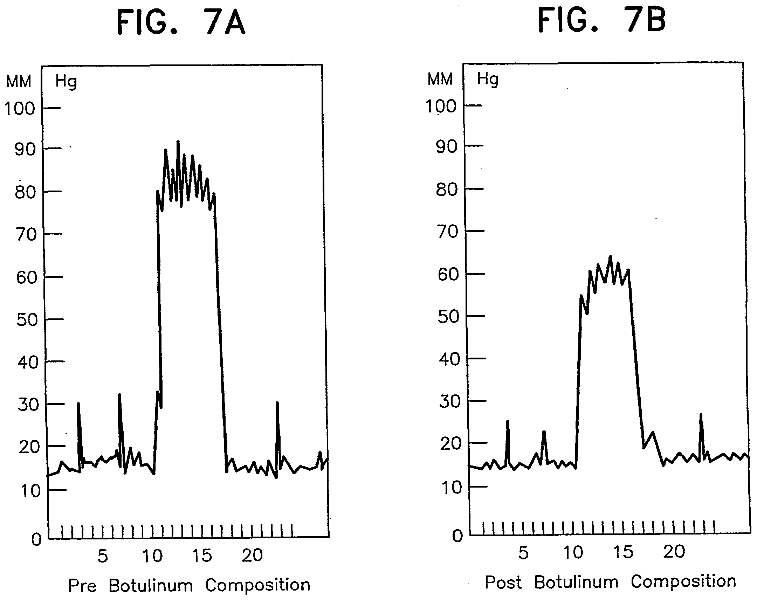

FIGS. 7A and 7B show graphs demonstrating greater and sustained increased of eye pressure during forceful squeezing of the eyelids, which is reduced after administration of botulinum toxin. The increase in eye pressure was maintained until the lids were open. X corresponds to time in seconds and Y corresponds to eye pressure in mm Hg. The graph of FIG. 7A shows squeezing the eyelids before administration of botulinum toxin (control). The graph of FIG. 7B shows squeezing the eyelids after administration of botulinum toxin. The higher and sustained spike observed pre-injection shown in the graph of FIG. 7A corresponds to higher eye pressure by the forceful closure of the eyes. Although the ability to squeeze the eye and increase eye pressure is preserved after injection of the compound as shown in the graph of FIG. 7B, the magnitude of the pressure spike during forceful closure is reduced when compared to the magnitude of pressure elevation in the graph of FIG. 7A (pre-injection). It was noted that the forceful squeezing of the eyelids after receiving Botulinum toxin lead to about a 50% lower increase in eye pressure (graph of FIG. 7B) when compared with the control as depicted in the graph of FIG. 5A.

Since Botulinum toxin can be relatively expensive and current vials come with a great amount of toxin (100 U per vial) and further considering that according to the principles of the invention the dosage of toxin is smaller than conventionally administered for blepharospasm and strabismus. A new vial containing smaller amounts of toxin was created. This will avoid wasting rather expensive toxin and will make therapy affordable and more widely used. Accordingly a new vial was designed containing 50 U (units) of dried Botulinum toxin type A. In this case reconstituting dried toxin with 8.0 ml of diluent (0.9% sodium chloride) results in 0.625 U of toxin per 0.1 ml. For advanced glaucomas 4.0 ml of diluent may be added to allow higher concentration, i.e., 0.625 U per 0.05 ml. In mild cases of glaucoma or with low pressure elevation during blinking a dose of 0.025 ml can be used corresponding to 0.3125 U of toxin. Those new arrangements and vials fit ideally with the new syringe with a capacity for 0.5 ml. The new syringe is subdivided and marked in 0.05 ml increments. It is understood that a variation of the dosage and arrangement for delivery of said dosage can be used without departing from the scope of the invention and principles disclosed herein.

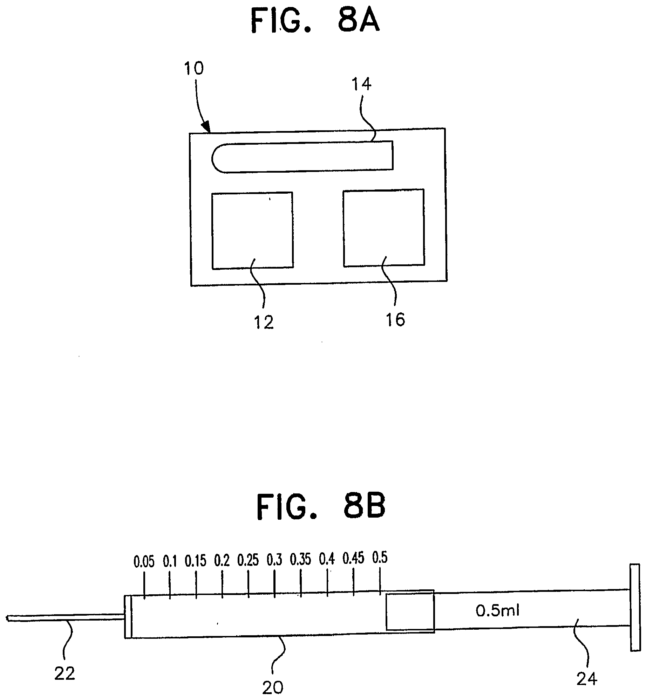

FIG. 8A shows the kit 10 containing one vial with dried toxin 12, one syringe with or without needle 14, and one vial with diluent 16. It is understood that the kit may contain two syringes and/or contain other needles (larger size) to aspirate the diluent. It is understood that vials with any amount of toxin can be used in the kit including a conventional vial with 100 U of toxin or the new vial with 50 U of toxin. A 27 to 30 gauge needle in the kit may be used to pierce the skin. A simpler kit may contain only the newly designed 0.5 ml syringe and one vial of 50 U of dried toxin.

FIG. 8B shows the newly designed syringe 20 subdivided in 0.05 ml increments for a total volume of 0.5 ml per syringe. The syringe 20 is connected to a hollow needle 22 which can pierce the target tissue. The syringe contains a piston 24 to press the fluid (toxin) thereby delivering said toxin through the needle 22 directly to the target tissue. Alternatively a dual needle system connected to a dual chamber syringe can be used allowing simultaneous delivery of toxin at two target sites (sites a and b in FIG. 1) in the eyelid margin. A further alternative to piercing the skin is to use a device which applies electrical current and to deliver the toxin and other compounds disclosed herein via iontophoresis, which consists of an enhanced transport via the skin using the driving force of an applied electric field. The device comprises a holding means to hold the toxin, preferably ready-to-use solution of botulinum toxin serotype B; means to apply electric current; and activating means to activate the current so that it releases the drug; wherein said holding means is in contact with the skin. The kit of FIG. 8A can also alternatively include a seringe 14, alcohol vial substituting the dried toxin vial 1 2 , and a lidocaine vial substituting the diluent vial 16 for preparing the lidocaine composition according to the principles of the present invention.

In accordance with this invention, pharmaceutically effective amounts of botulinum toxin can be administered alone to treat glaucoma. Alternatively, the toxin compound may be administered sequentially or concurrently with another drug, e.g., injectable anesthetic, calcium channel blocker, or a glaucoma medication. The most effective mode of administration and dosage regimen of the combination toxin compound and drug will depend upon the the severity and course of glaucoma, previous therapy, the patient's health status, response to drug effect and judgment of the treating physician.

In order to optimize the therapeutic result a device, method and system were developed. The method also includes measuring the force of the lid or the pressure applied by the eyelid to the eye during blinking (involuntary or forceful) or squeezing the eye using LMDs, for example devices described by Abreu or other means and devices including any device capable of measuring the aforementioned external pressure effects or force of the muscles or force of the eyelid. The method more specifically includes the step of measuring pressure and/or force caused by external factors, and a second step that quantifies the amount and/or type of the drug to be applied based on levels identified in the first step. The first step can also include measurement of the elasticity of the muscles and/or eyelid distractibility and/or eyelid laxity. A clamp placed on the upper eyelid lashes and attached to a force transducer can also be used in the first step.

More specifically the methods include:
1). Method for non-invasively measuring eye pressure increase caused by eyelid muscle action (blinking) including the steps of: applying a contact lens-like pressure measuring device to the eye, inducing a change in said pressure measuring device by moving the upper eyelid down to cover said pressure measuring device, sensing the eye pressure at the exact time the eyelid interacts with said pressure measuring device, and determining the change in eye pressure caused by the blinking action with a signal received from said pressure measuring device. These steps allow obtaining a baseline of the pressure effect of the lid during blinking. This gives information of how much eye pressure increase or eye pressure spike occurs due to muscle action during blinking. Subsequent to these steps, the next step includes quantifying the amount of compound required to reduce the spike during blinking based on the level of pressure spike acquired in the previous step. This method allows precise quantification of the compound chosen according to the individual needs of the patient and target pressure determined by the doctor treating said patient.
2). Method for non-invasively measuring eye pressure increase caused by eyelid muscle action (squeezing the eye) including the steps of: applying a contact lens-like pressure measuring device to the eye, inducing a change in said pressure measuring system by forcefully moving the upper eyelid down to cover said pressure measuring device, squeezing the eyelids, sensing the eye pressure with said pressure measuring device, and determining the change in eye pressure caused by the squeezing action with a signal received from said pressure measuring device. These steps allow obtaining a baseline of the pressure effect of the lid during squeezing the eyes. This gives information of how much eye pressure increase or eye pressure spike occurs due to muscle action during squeezing the eye. Subsequent to these steps, the next step includes quantifying the amount of compound required to reduce the spike during squeezing of the eyes based on the level of pressure spike acquired in the previous step. This method allows precise quantification of the compound chosen according to the individual needs of the patient and target pressure determined by the doctor treating said patient.
3). Method for evaluating the therapeutic effect of lowering eye pressure by modulating muscle action including the steps of: applying a contact lens-like pressure measuring device to the eye, inducing a change in said pressure measuring system by forcefully moving the upper eyelid down to cover said pressure measuring device, squeezing the eyelids, sensing the eye pressure with said pressure measuring device, and determining the change in eye pressure caused by the squeezing action with a signal received from said pressure measuring device, applying a chemical composition based on the levels of eye pressure identified in the previous step, reapplying the contact lens-like pressure measuring device to the eye, inducing a change in said pressure measuring device by forcefully moving the upper eyelid down to cover said pressure measuring device, squeezing the eyelids, sensing the eye pressure with said pressure measuring device, and determining the change in eye pressure caused by the squeezing action with a signal received from said pressure measuring device to determine if the desired therapeutic effect has been achieved.

In summary the method includes two basic steps and an optional third step. First step: determining a baseline of the pressure effect of the lid and facial muscle activity. This gives information of how much eye pressure increase or eye pressure spike occurs due to muscle action and muscle tension. Subsequent to the first step, the second step includes the use of drugs to modulate, blunt, or alter the muscle activity identified in the first step, by for example administering injections of lidocaine composition or botulinum toxin to the eye and/or facial muscles. After the administration of therapy a third optional step includes the use of LMD to measure the effect of the injection, or alternatively the effect is measured 3 to 5 days after the injection, to make sure the therapeutic effect needed and lowering of eye pressure was achieved. This allows precise individual therapy according to the needs of the patient.

Preferably, a potentially lower (0.5 U) than the optimal dose is administered to identify the therapeutic effect and side effect profile. If the LMD determines that the therapeutic effect has been achieved, then the therapy is finished. If LMD determines that further pressure lowering is necessary, another dose is then applied. The exact amount of toxin can be delivered to keep the eye pressure below the target pressure and according to the state of progression of the disease. After this baseline and dose response is achieved, therapy can be done in one simple step of injecting a similar amount of medication as identified before providing that adequate therapeutic effect is achieved and the eye pressure level is acceptable in reference to the target pressure.

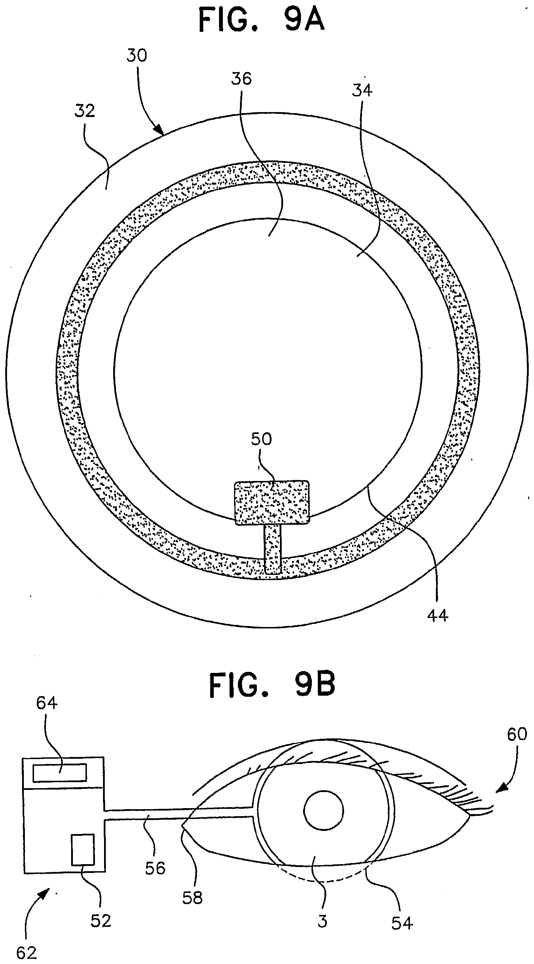

Although the invention can be practiced without the need of using LMDs, such as by measuring eye pressure with a standard tonometer and using an universal dosage of the compound for each type of the disorder, said LMDs can provide a numerical value of the actual EPE. Accordingly, as illustrated in FIGS. 9A and 9B, an exemplary LMD 30 is provided for measuring EPE. FIG.9A is a schematic planar view showing the contact lens-based LMD 30. The LMD system 30 includes a contact device 32 for placement in contact with the cornea, and a central cavity 34 containing fluid 36. The central cavity 34 is disposed within the contact device 32 and includes a wall 44 made with a flexible and distensible material such as silicon rubber used for making contact lenses. With eye closure, cavity 34 with fluid 36 is deformed and the inferior portion of said cavity 34 projects inwardly against the cornea increasing the pressure inside said cavity 34 which is in physical equilibrium with the intraocular pressure. Cavity 34 contains a radio frequency (RF) pressure sensor microchip 50 adapted to provide a signal corresponding to the IOP level due to EPE. Microchip 50 measures pressure and contains the processing and transmitting capabilities to generate a signal corresponding to the increase in eye pressure caused by the eyelid 1 and to transmit the signal to a remote receiving unit. Microchip sensor 50 can be ultra-thin and the pressure sensor, support circuitry, RF power and communications are all deposited on a micro-chip die allowing the circuit to be built in large quantities and at very low cost.

FIG. 9B shows an alternative embodiment in which the pressure sensor 52 is located away from the contact device 54. FIG. 9B is a schematic frontal view of contact device 54 laying on the cornea 3 with conduit 56 exiting the eye 60 at the lateral medial corner of the eye 58 and connecting with sensing unit 62 which contains pressure sensor 52 and the necessary processing and display means 64 to process and display the IOP value measured. Conduit 56 can work as a conduit for fluid from contact device 54 to sensing unit 62 which are in physical equilibrium, thus the pressure measured on cornea 3 by contact device 54 is reproduced in sensing unit 62. Alternatively, conduit 56 can work as a microwire connecting a pressure sensor disposed within the contact device 54 to an external reader.

The preferred embodiment shown in FIG. 9A allows doctors to easily measure the EPE and effects of therapy using a completely unobtrusive, disposable, and low cost device. However, the alternative embodiments in FIG. 9B allows universal and easy construction of an EPE measuring unit using widely available and low cost discrete components.

The following example shows that even a patient with baseline IOP below target pressure is at risk of blindness due to increase IOP levels above the target pressure due to EPE. For example a glaucoma patient needs a target pressure of no higher than 21 mm Hg based on the health status of the optic nerve and visual field. That means that for this particular patient an eye pressure higher than 21 mm Hg can lead to visual loss and blindness. This patient is fully medicated with eye drops and the baseline eye pressure measured by conventional means is 18 mm Hg, which at first appears to be acceptable since it is lower than the target pressure of 21 mm Hg.

However in reality this patient is having eye pressure well above the target pressure as measured by the LMDs during muscle activity. This patient is thus at risk of going blind despite being fully medicated with eye drops with a baseline pressure below the target eye pressure. The reason is that during blinking as measured by LMD the eye pressure is reaching levels of 28 to 30 mm Hg, and over 90 mm Hg during squeezing or squinting of the eye. Since one blinks normally an average of 16,000 times daily, it means that this patient is being hammered thousands of times a day with pressures of 28 to 30 mm Hg which are well above the safety levels of 21 mm Hg needed to preserve vision. By using the method described the doctor evaluated the spike in pressure during blinking as 30 mm Hg and based on that information said doctor administered 1.5 U of botulinum toxin. The doctor tested the muscle effect on pressure three days later and noted that the spike in pressure was 25 mm Hg. Another 1 U of botulinum toxin was then injected which after evaluation with LMD showed to be 20 mm Hg, which is an acceptable level below the target eye pressure of 21 mm Hg.

Corneal curvature is modified by the position or tension of the eyelids. The elastic coefficient of the lids correlates with the amount of lid tension. In people with normal eyelids the elastic coefficient of the lid is about 3.22 g/mm. It is important to measure eye pressure with LMD and to evaluate the physical properties of the lids in order to deliver the amount of medication needed to modulate the effect of the muscle tension for each patient individually. The tension applied by the lid to eyes with glaucoma causes an increase in eye pressure that can be controlled with the therapeutic modalities of the present invention. The elasticity of the lid and/or pressure caused by the lid can be measured to precisely quantify the decrease in eye pressure that has been achieved after therapy is applied to the eye. Therapy is administered to reduce the muscle tension effect of the eyelid causing increased IOP.

The methods described above can be used to enhance the outcome of eye surgery comprising the step of measuring eyelid muscle function and then applying medication to control said eyelid muscle function in order to reduce pressure spikes after surgery.

The degree of blockade of muscle activity and eyelid pressure can be regulated by variation of dosage, variation in the sight of injection, and frequency of injection. Higher dosages or lower dosages can be employed provided the dose is effective in reducing muscle activity and eye pressure spikes while being non-toxic and preserving normal eyelid function. Some patients who maybe resistant to the toxin or who have developed antibodies may require injections of more than 10U per site. The injections are preferably performed in the pretarsal area of the eyelids using between 0.5U and 15 U of botulinum toxin A per site, most preferably using two sites adjacent to the Riolan muscle. The method also includes changing to a different serotype of toxin in case of antibody formation.

Glaucoma patients using drugs that increase blinking and blepharospasm such as neuroleptics (e.g: trifluoperazine, phenothiazines) and antihistamines can benefit from prophylatic use of drugs to modulate muscle activity as disclosed herein. Other glaucoma patients who would particularly benefit from the therapy include patients with moderate to marked dermatochalasis, large volume lesions in the lid, and with subjective heaviness of the eyelids as well as patients who underwent upper-eyelid blepharoplasty. Another group of patients that can particularly benefit from the therapy disclosed herein are patients with pigmentary glaucoma and pigment dispersion syndrome. An individual without glaucoma but with family history of blindness due to glaucoma can benefit from the prophylatic use of the compounds of the present invention, even before said individual is diagnosed with glaucoma.

Polymyography and electromiograms are not necessary but can be used to guide the treatment with botulinum toxin and to monitor the muscles to be injected.

A further advantage is that since the therapy is physician based and the preferred embodiments do not require eye drops, patients can enjoy life instead of having to apply eye drops to the eyes, which is a time consuming task that has to be done daily, and even several times a day.

### Modulation of Muscle Activity and Neuro-Protection

Other compounds that are disclosed herein include calcium antagonists, gabapentin or other agents that decrease muscle activity and act as a muscle relaxant. Calcium anatagonists act primarily in non-skeletal muscle but have the advantage of also having neuroprotective effects like botulinum toxin and gabapentin. Those calcium antagonists are injected in the eye muscles in a similar manner in accordance with the principles of the invention in order with modulate muscle action and provide neural protection.

The compounds can work as neuroprotective agents, which protect the nerve by acting preferably as antagonists of glutamate or NMDA. In vitro studies have indicated that botulinum toxin inhibits the evoked release of glutamate in primary cultures of spinal cord neurons and that in brain synaptosome preparations botulinum toxin inhibits the release of both neurotransmitters, acetylcholine and glutamate.

Thus botulinum toxin may be used for treatment of retinal ganglion cell damage associated with glaucoma, optic neuritis, optic neuropathy, or any other disease that leads to neuronal injury mediated by glutamate. The therapy with the toxin may be done in combination with other modes of treatment, e.g., those that are directed to reducing intraocular pressure. The toxin can act as a neuroprotective agent and may be given to any patient at risk of neuronal injury in a similar manner as described for glaucoma, but in lower doses, preferably half of the dose, and more frequently. The toxin in association with other neuroprotective agents such as amantadine can act to prevent cell death and be used in combination with other neuro protective agents. Any neuroprotective agent that acts on N-Methyl-D-aspartate (NMDA) receptor or on glutamate as well as any antagonists
of excitatory amino acid receptors both NMDA and non-NMDA subtypes) can be used in conjunction with the toxin. The reduction of excitotoxicity can also be used to treat optic neuritis by inhibiting glutamate mediated cell damage. For example, ketamine is a N-Methyl-D-Aspartate Antagonists and gabapentin has affinity and activity at NMDA receptors.

area compound according to the invention is botulinum toxin. This compound is to be administered by percutaneous injection in the musculature at the eyelid margin adjacent to the muscle of Riolan.

Gabapentin is one of the preferred compounds for glaucoma therapy because gabapentin stabilizes neuronal membranes and modulates the eyelid muscle activity and also acts as a glutamate antagonist. Gabapentin (available from Pfizer, NY under the trade name NEURONTIN) is an anticonvulsivant agent structurally related to the inhibitory CNS neuro transmitter gama-aminobutiric acid (GABA). Gabapentin achieved therapeutic effect taken as tablets by mouth at a daily dosage of 800 mg to 1200 mg, although lower or higher doses may be needed depending on clinical aspects.

### Modulation of Muscle Activity and Enhancement of Blood Flow

Another aspect of the invention features enhancement of blood flow to the retina and optic nerve. The method for maximizing the health of the optic nerve and retina in eye disorders by increasing retinal and optic nerve blood flow comprises applying to the eye an effective amount of a compound that reduces external pressure effects on the eye in an amount effective to effect such reduction, thereby alleviating the pressure inside the eye, and by alleviating the pressure inside the eye allowing a larger blood volume to reach the eye. Perfusion pressure is key for maintaining proper blood supply to the retina and optic nerve.

The higher the IOP the lower the perfusion pressure which is translated in lower blood flow to the eye. Blood flow to any tissue is the result of perfusion pressure which is basically the arterial pressure minus venous pressure and the resistance to flow between arteries and veins. In the eyes a third component, IOP, plays a key role. IOP challenges blood flow to the eye by raising venous pressure at the exit point which in turn lowers perfusion pressure. For a vein to remain distended, pressure in the lumen of the vein must exceed that of surrounding tissue, so within the eye, venous pressure must be above IOP for flow to exist. The arteriovenous pressure difference is reduced when venous pressure is elevated due to elevated intraocular pressure. Therefore, if IOP is 20 mm Hg instead of 14 mm Hg, the venous pressure has to rise to become higher than 20 mm Hg, which is accomplished by venous constriction. If the venous pressure cannot be raised to surpass IOP, ischemia (lack of oxygen) ensues which can lead to damage to the eye. Since IOP even in normal eyes is higher than orbital venous pressure, perfusion pressure in an eye with normal IOP is already less than in other tissues, and is reduced even more if IOP becomes further elevated. EPE causes increase in IOP that can lead to ischemia and even precipitate visual loss in certain conditions. The impact on blood flow due to IOP increases in proportion to the increase in IOP, up to the point of cessation of blood flow when the IOP equals mean arterial pressure. EPE can lead to such great magnitude of IOP increase that can significantly reduce blood flow to the eye, and even transiently stop blood flow to the eye. Normal and young people have mechanisms of autoregulation that increase flow by decreasing resistance. However, in certain systemic diseases, eye disorders, and with increasing age ischemia and damage to the eye can occur due to reduced blood flow.

This potential lack of oxygen is particularly significant in two situations.
1. When the capacity of the regulatory mechanism has been exceed, as for example when the IOP is very high, such as due to EPE. This is of medical significance in patients with high cholesterol and high blood pressure. In those cases the presence of atherosclerosis has caused the autoregulatory capacity to be already utilized, therefore little is left to respond to the additional challenge caused by increased IOP due to EPE.
2. When the regulatory mechanism becomes defective or less responsive, as occurs in eye disorders, systemic diseases and aging. For example, in diabetes the autoregulatory mechanism is less responsive, and in patients with vascular disease or atherosclerosis the mechanism becomes defective since the wall of the blood vessels are hardened by atheromas (fat deposit). Patients with any of the eye disorders mentioned such as glaucoma who also are afflicted with high cholesterol, high blood pressure, diabetes, or heart disease may be more susceptible to damage to the eye due to EPE, and therefore the EPE should be treated as early as possible to avoid unnecessary damage and visual loss.

Since the retina has decreased oxygenation and decreased blood perfusion in diabetic retinopathy, vascular occlusions, optic neuropathy, macular diseases and other conditions, any increase in eye pressure can lead to a decrease in perfusion pressure and reduction of blood flow to said retina. The increase in eye pressure hampers and slows down the arrival of blood to the retina. The frequent (16,000 times daily) and substantial (increase of eye pressure by 20 mm Hg, and up to over 100 mm Hg during squeezing the eyelids) eye pressure elevation caused by external pressure effects can cause substantial reduction of blood flow in the abnormal capillary bed and vasculature as found in diabetic retinopathy, central retinal vein occlusion, central retinal artery occlusion, ischemic optic neuropathy, retinitis, macular edema and macular degeneration. In glaucoma there is also a reduction of blood flow to the optic nerve and the remaining blood flow to said optic nerve can be further reduced by the increase in eye pressure caused by the external pressure effects.

By reducing eye pressure caused by external pressure factors, there is enhancement of blood flow and perfusion pressure to the retina and optic nerve. Thus the present invention can be used to increase blood flow to the retina and to the optic nerve and be used to treat any condition that can benefit from better retinal oxygenation or better blood flow to the retina and/or optic nerve. Exemplary conditions include Diabetic retinopathy, Retinal vein occlusion, Retinal artery occlusion, Anterior ischemic optic neuropathy, Hypertensive retinopathy, Sickle-cell retinopathy, Macular Edema and Age-related macular degeneration

### Modulation of Muscle Activity and Reduction of Muscle Volume and Muscle Contraction

It is understood that other agents such as myotoxins (e.g.: doxorubicin) can be used according to the principles disclosed herein. The use of myotoxins (d rug which have a toxic effect on the muscle) such as doxorubicin and the like can be injected in the eyelid and facial muscle in a similar manner as botulinum toxin according to the principles disclosed herein. Doxorubicin promotes a chemomyectomy and injections of 0.5 mg to 1 mg of free doxorubicin have proven to be effective for reducing and modulating both muscle volume and muscle contraction.

The reduction in volume may be particularly beneficial to patients with increased volume of muscles as occur in thyroid eye disorder and Grave' s disease. The description discloses a novel therapy for reducing size of muscles with direct injection of the drug into the eye muscles including extraocular muscles.

Decrease in muscle contraction can also be achieved with a myotoxin and in thisregard, the drug is applied preferably to the eyelids for treating glaucoma and other eye disorders which can benefit from at least one of reduced eye pressure, reduced muscle volume and increased blood flow to the eye.

An exemplary protocol includes injection of Doxil (Sequus Phrmaceuticals, Menlo Park, CA) at a dose of 0.1 mg in a volume of 1 ml saline into the eyelid region preferably adjacent to Riolan's muscle and/or pre-tarsal orbicularis muscle. Pretreatment includes injection of a mixture of 0.75% bupivacaine (Sensorcaine; Astra Pharmaceuticals, Westborough, MA) containing 1:200,000 epinephrine and hyaluronidase (150 units, Wydase; Wyeth Laboratories, Philadelphia, PA) in 1 ml, 30 minutes before administration of Doxil into that eyelid. It is understood that that dose per treated eyelid ranging from 0.5 to 3.0 mg of free doxorubicin can be used depending on the clinical need of the patient. A preferred dose of 0.5 mg of doxorubucin HCl (Adriamycin, available from Pharmacia Corporation, Peacock, NJ) in 1 ml of sterile isotonic saline solution injected into the eyelid muscle can have a similar effect. Our studies have shown that direct, local injection of doxorubicin, either free in solution or in liposome-encapsulated form, can have a modulating therapeutic effect on eye muscles and reduce the strength of eye muscles, and be used in therapy of glaucoma and other eye disorders that benefit from reduced eye pressure.

### Modulation of Muscle Activity for Use in Anterior Segment Disease, Cataract Surgery and for Improving Surgical Outcomes

The present description also provides methods for the therapy of corneal disorders or to preserve corneal epithelium. The corneal epithelium can be displaced by the forces applied to the cornea due to external factors. By blunting the effect of said forces the epithelium is preserved such as in cases where there is an epithelial defect or injury to the cornea. Many of the anterior surface diseases of the eye are worsened by lid action and by disruption of the anterior surface by the pressure applied by the lid. Any anterior surface disease can be treated in accordance with the present invention with reduction of the lid effect with preservation of the anterior surface. Exemplary anterior diseases that can benefit from the therapy include corneal degeneration, corneal dystrophy, corneal ulcers, ocular herpes, corneal edema, bullous keratopathy, conjunctivitis, scleritis and the like.

One particularly case in which modulating lid muscle force is particularly useful is in refractive surgery. One of the problems in refractive surgery is the patient squeezing their eyes during the procedure. By using the compositions disclosed herein, said eyelid forces are modulated preventing squeezing of the eye during the procedure. In addition, after the procedure, epithelial healing and flap healing are critical for visual preservation. By modulating the forces of the eyelid there will be much less impact on the healing process of the corneal epit*µ*elium or the flap created during surgery, besides reduced chance for flap slippage.

The present description also provides methods for matching measurements of corneal topography before and during refractive. The eyelids are in contact with the corneal surface in normal viewing and the lids distort corneal shape. The eyelids can affect the natural topography surface of the eye. Refractive surgery, however, is performed with a speculum in place and thus the lids are not in contact with the cornea. The speculum effectively prevents any lid contact with the cornea. Preoperative testing (e.g.: topography) is done with eyelids in contact the corneal surface, but the surgery is performed with a speculum in place that removes the lid forces.

Accordingly, if the presence of the lid effect induces a shape change on the cornea, removing the lid force will cause the cornea to have another shape during the surgery. To equalize the corneal surface in the pre-operative testing with the corneal surface during surgery the lid effect should be removed during measurements before surgery. By applying the compounds disclosed herein the lid effect is removed. The same shape of the cornea that will be used during surgery can then be measured pre-operatively using topography. The laser can calculate its ablation based on the topography. Since the topography is equal, before and during surgery, the most precise ablation can be achieved.
This method is key when using wave-front technology in refractive surgery. By modulating muscle effect in accordance to the principle disclosed herein the postoperative ablation results becomes predictable as the forces applied by the lid and the eye pressure were known and the surface of the cornea is equalized before and during surgery by applying the chemical compositions disclosed herein.

The method also includes using a muscle relaxant such as botulinum toxin or lidocaine composition before the surgery in a way that the motion of the lid is reduced while preserving muscle tension in order to match the shape of the cornea before and during the procedure, in a manner that the use of a speculum may not be necessary since motion of the eyelid during surgery has been modulated.

Patients with glaucoma undergoing a glaucoma operation, cataract operation, retina surgery, cornea surgery, plastic surgery, or any other eye surgery can benefit from preoperative treatment with the compounds with reduce EPE in accordance with the present invention. A glaucoma patient can lose vision or even go blind after eye surgery because of increased eye pressure in the post-operative period. The
external pressure effects could work as the trigger for precipitating blindness. By treating and decreasing those external pressure effects there is a much lower chance of vision loss post-operatively.

### Modulation of Muscle Activity and Treatment for Facial Wrinkles

Considering the successful muscle relaxation achieved, patients underwent treatment for reduction of facial wrinkles. A 56 year old patient received 0.3 ml of a lidocaine composition comprised of 2 ml of lidocaine O. 5% added to 99.5% ethanol in one tenth of the volume of lidocaine. The therapy with the lidocaine composition according to the principles and dosage disclosed in the present description lead to a reduction of wrinkles in said patient after injection along frown lines. Thus the lidocaine composition can treat muscle over contraction which create wrinkles. The therapy provided may offer some advantages over currently used botulinum toxin, since the lidocaine composition is low cost, safe and the injection is painless since the therapy uses an anesthetic in its composition. Contrary to that the injection of botulinum toxin can be painful and involves an expensive compound.

The lidocaine composition acts by relaxing the muscle and making the wrinkle disappear. The present description also provides a safe, effective, painless and much lower cost therapy for treating wrinkles and other muscle disorders using the compounds disclosed in the present invention, most preferably using an amide type anesthetic such as lidocaine combined with alcohol, alone or in conjunction with other compounds. The compounds disclosed herein can also be used as a substitute to treating disorders in which botulinum toxin is used including current and future uses of botulinum toxin. The dosage of botulinum toxin can also be reduced by combing said botulinum toxin with the amide-type anesthetic. Exemplary amide type anesthetics include lidocaine, bupivacaine, mepivacaine and ropivacaine.

### Other Compounds for Modulating Muscle Activity

Other compounds that reduce or modulate eye muscle activity and are useful include:
mexiletine, trihexyphenidyl, baclofen, clonazepam, tetrabenazine, cannabinoids (tetrahydrocannabinol, cannabidiol and cannabigerol), benzodiazepines, tiapride, benztropine mesylate, clozapine, quinine, anti-dopaminergics, imipramine and other anti-depressant drugs and the like. Baclofen is a stable analog of GABA and interacts primarily with the inhibitory GABA-B receptors. Mexiletine can be used at a dose of 50 mg to 100 mg, once to three times a day, up to 600 mg a day, taken by mouth.

Other compounds that can be injected in a similar fashion as botulinum toxin and anesthetics (e.g. : lidocaine, bupivicaine, and ketamine) include amantadine derivatives, nitroglycerin derivatives, phenol, and the like. Local injection of verapamil (0.1 to 1.8 mg) in the manner described also causes desired therapeutic effect. Verapamil can also be injected prior to the injection of botulinum toxin or doxorubicin in order to decrease the amount of botulinum toxin or doxorubicin administered. The combination of at least one of hyaluronidase, collagenase, curare agents and bupivacaine can be used alone or in combination with botulinum toxin. The blockade of the superior sympathetic ganglion with local anesthetic also can provide the modulation of eyelid muscle force. Topical nitrates alone can be used to decrease eye pressure via acting at both internal and external pressure effects.

Botulinum toxin action can be enhanced by concomitant use of nitrates or aminoglycoside antibiotics, the latter can induce an added alteration of neuro transmission at the presynaptic level. 100 mg of lidocaine injected intravenously over five minutes also promotes the desired effect, but the effect is short lived and is too risky due to potential cardiac side-effects. It is understood that a variety of combinations of drugs injected or taken by mouth described herein can be used in accordance with the principles presented herein to modulate muscle action and decrease EPE.

Any of the chemical compositions disclosed herein to modulate external pressure effects can be injected adjacent to the muscle, directly into the muscle or nerve, being delivered via liposome- encapsulated form, implanted, and the like. Applicatiuons of muscle relaxants as tablets by mouth can be done alone or in sequence with other agents applied directly into the ocular and periocular muscles, such as lidocaine composition, botulinum toxin, conventional eye drops to treat glaucoma, and the like.

As described above, the inventive compounds are administered by percutaneous injection, means not according to the invention including topically as an eye drop, orally as tablets as well as liquid excipients, suspensions, and patch applied to the skin of the eyelids for the delivering of the drug over time. The patches can have chemical enhancing means to increase penetration of the drug, or have micro-needles that deliver the medication, but it is not felt by the patient. In addition, botulinum toxin in the form of eye drops, such as a ready-to-use solution as provided by serotype B, can be used to decrease eye pressure by acting inside the eye and increase the rate of filtration through the drainage system and decreasing production. Botulinum toxin can effectively reduce internal pressure effects when administered as a 0.01 to 5% solution in an opthalmologically acceptable carrier, or preferably administered as a 0.5 to 2% solution in an opthalmologically acceptable carrier. Furthermore, injections of botulinum toxin into the subconjunctival space at the superior margin of the tarsal plate, via a conjunctival approach, as well as intravitreally can also be used to reduce eye pressure and increase blood flow. The drugs described herein may be compounded into a pharmaceutical preparation, using pharmaceutical compounds well-known in the art. Those skilled in the art will appreciate how to formulate acceptable drug delivery means, formulations and therapeutics.

Although some preferred doses were disclosed herein, it is understood that doses can vary significantly according to the clinical condition of the patient and type of botulinum toxin used. For example, the two commercially available botulinu toxin type A differ significantly in their dose and composition. Botox (Allergan, Irvine, CA and Dysport (Ipsen and Porton) of United Kingdom) are supplied in vials with 100 and 500 Units of toxin respectively, with an equivalence relationship of one Botox unit corresponding to approximately three to five units of Dysport.

The compositions used in these therapies may also be in a variety of forms. These include, for example, solid, semi-solid, powder, and liquid dosage forms or suspension, liposomes, injectable and surgically implantable means. The preferred form depends on the intended mode of administration and therapeutic application. The compositions also preferably include conventional pharmaceutically acceptable carriers.

It is understood that any compounds by any route of administration can be used as long as they are capable of reducing or modulating muscle activity, blinking, or squeezing the eyelids or reducing the possibility of pressing the eyelids against the eyeball including all of the maneuvers and muscle action which can cause an increase in intraocular pressure that can be prevented or treated including physical barriers such as wearing specialty goggles for sleeping. It is understood that any form of chemodenervation and/or chemomyectomy therapy can be used.

The toxin agent and other drug can be administered simultaneously or sequentially with the other drug being administered before, after, or both before and after treatment with the toxin. Conventional modes of administration and standard dosage regimens of botulinum toxin agents described herein can be used, however smaller doses may be used if the other drug is providing a good control of the disease. Optimal dosages for coadministration of a drug with a toxin agent can be determined using methods known in the art. Dosages of toxin agents may be adjusted to the individual patient based on the dosage of the drug with which the toxin agent is co-administered and by the response of the patient to the treatment regimen. As mentioned, botulinum toxin may be used to reduce or prevent glaucoma related injury to the retinal ganglion cells and their axons comprising the optic nerve. The toxin agent, lidocaine composition, and other compounds disclosed herein can also be used in combination with other eye drops (e.g.: artificial tears) as well as glaucoma agents in the form of eye drops, pills, implants, surgical devices and the like. Those skilled in the art will appreciate how to formulate acceptable therapeutics and combination therapeutics. Various biological targets for modulating muscle action are included herein. Accordingly exemplary targets and an exemplary compound acting on said target includes: sodium channel (e.g., lidocaine), acetylcholine receptor in presynaptic membranes (e.g., botulinum toxin), dopamine receptor (e.g., lisuride), GABA receptor (e.g., clonazepan and Baclofen), muscarinic receptor (e.g., Trihexyphenidyl) and calcium channel (e.g., verapamil). In view of the discovery that external pressure effects cause a significant increase in eye pressure of glaucoma patients and ocular hypertensives and reduce blood flow to the eye, and that muscle relaxants can decrease the eye pressure and increase blood flow, any suitable muscle relaxant injectable, oral, or topical acting locally in the muscle vicinity, in the peripheral nerve, or centrally (central nervous system) may be used, and exemplary agents are disclosed in Table 1.

| Table 1 |
|---|
| AGENTS |
| Lidocaine |
| Bupivicaine |
| Amide anesthetics |
| Botulinum toxin |
| Tetanus toxin |
| Tetrodotoxin |
| Funnel web spider venom |
| Saxitoxin |
| Ethanol |
| Phenol |
| Doxorubicin |
| Gabapentin |
| Mexiletine |
| Trihexyphenidyl |
| Verapamil |
| Hyaluronidase |
| Collagenase |
| Ketamine |
| Curare agents |
| Baclofen |
| Aminoglycosides |
| Clonazepam |
| Tetrabenazine |
| Tetrahydrocannabinol |
| Cannabidiol |
| Cannabigerol |
| Calcium channel blockers |
| Benzodiazepines |
| Clozapine |
| Tiapride |
| Benztropine mesylate |
| Selegiline |
| Quinine |
| Anti-dopaminergics |
| Imipramine |
| Lisuride |
| Amantidine derivatives |
| Nitroglycerin |
| Nitrates derivatives |
| Lidocaine and ethanol |
| Bupivicaine and ethanol |
| Botulinum toxin and ethanol |
| Bupivicaine and verapamil |
| Lidocaine and verapamil |
| Botulinum toxin and verapamil |
| Composition using 2 of the above compounds |
| Composition using 3 of the above compounds |
| Composition using 4 of the above compounds |

## Claims

1. An effective amount of a compound that reduces external pressure effects for use in treating glaucoma in a mammal, wherein external pressure effects experienced by the mammal are alleviated,
wherein said compound is
a) botulinum toxin; or
b) a combination of lidocaine and alcohol;
and wherein the compound is administered by percutaneous injection into one or more points in the musculature at the eyelid margin adjacent to the muscle of Riolan.

2. Compound for use according to claim 1, **characterized in that** the botulinum toxin is selected from the group consisting of botulinum toxin types A, B, C, D, E, F and G.

3. Compound for use according to one of the claims 1 to 2, **characterized in that** it is applied during or after refractive surgery of an eye having glaucoma.

4. Compound for use according to one of the claims 1 to 3, **characterized in that** it is used in combination with eye drops to treat glaucoma of a patient.

5. Compound for use according to claim 4, **characterized in that** the eye drop is a prostaglandin analogue.

## Patentansprüche

1. Wirksame Menge einer Verbindung, die externe Druckwirkungen reduziert, zur Verwendung in der Behandlung von Glaukom in einem Säugetier, wobei von dem Säugetier empfundene externe Druckwirkungen gelindert werden, wobei besagte Verbindung
a) Botulinumtoxin; oder
b) eine Kombination von Lidocain und Alkohol;
ist und wobei die Verbindung über perkutane Injektion in einen oder mehrere Punkte in der Muskulatur am Rand des Augenlids benachbart zum Riolan-Muskel verabreicht wird.

2. Verbindung zur Verwendung gemäß Anspruch 1, **dadurch gekennzeichnet, dass** das Botulinumtoxin ausgewählt ist aus der Gruppe bestehend aus Botulinumtoxin A, B, C, D, E, F und G.

3. Verbindung zur Verwendung gemäß einem der Ansprüche 1 bis 2, **dadurch gekennzeichnet, dass** sie während oder nach einer refraktiven Chirurgie eines Auges mit Glaukom appliziert wird.

4. Verbindung zur Verwendung gemäß einem der Ansprüche 1 bis 3, **dadurch gekennzeichnet, dass** sie in Kombination mit Augentropfen verwendet wird, um ein Glaukom eines Patienten zu behandeln.

5. Verbindung zur Verwendung gemäß Anspruch 4, **dadurch gekennzeichnet, dass** die Augentropfen ein Prostaglandin-Analogon sind.

## Revendications

1. Quantité efficace d'un composé qui réduit des effets de la pression extérieure, destiné à une utilisation dans le traitement du glaucome chez un mammifère, dans lequel des effets de la pression extérieure subis par le mammifère sont réduits ;
dans lequel ledit composé est :
a) une toxine botulinique ; ou
b) une association de lidocaïne et d'alcool ;
et dans lequel le composé est administré par injection percutanée en un ou plusieurs points dans la musculature au niveau du bord de la paupière adjacent au muscle de Riolan.

2. Composé destiné à une utilisation selon la revendication 1,
**caractérisé en ce que** la toxine botulinique est sélectionnée dans le groupe constitué par des toxines botuliniques de types A, B, C, D, E, F et G.

3. Composé destiné à une utilisation selon la revendication 1 ou la revendication 2, **caractérisé en ce qu'**il est appliqué pendant ou après une chirurgie réfractive d'un oeil qui présente un glaucome.

4. Composé destiné à une utilisation selon l'une quelconque des revendications 1 à 3, **caractérisé en ce qu'**il est utilisé en association avec des gouttes ophtalmiques de façon à traiter un glaucome chez un patient.

5. Composé destiné à une utilisation selon la revendication 4,
**caractérisé en ce que** la goutte ophtalmique est un analogue de prostaglandine.
